# EUROPEAN PATENT APPLICATION

(11) **EP 4 187 551 A1**
(43) Date of publication of application: **31.05.2023**
(21) Application number: 22210086.9
(22) Date of filing: 29.11.2022
(51) Int. Cl.: G16H 50/20, G16B 20/50, G16H 70/20, G16H 70/60

(54) **REPORT GENERATING METHOD OF GENERATING A REPORT REPORTING A LEVEL OF PATHOGENICITY OF A GENETIC MUTATION, AND A REPORT GENERATING DEVICE**

(30) Priority: 30.11.2021 JP 2021194091
(71) Applicant: SYSMEX CORPORATION, Kobe-shi Hyogo 651-0073 (JP)
(72) Inventor: Izumi, Yuuki, Kobe-shi, Hyogo, 651-0073 (JP); Niiro, Hayato, Kobe-shi, Hyogo, 651-0073 (JP); Washio, Takanori, Kobe-shi, Hyogo, 651-0073 (JP)
(74) Representative: Hoffmann Eitle

(57) **Abstract**

A report generating method generates a report for reporting a level of pathogenicity of a genetic mutation. The report generating method comprising obtaining information on a genetic mutation detected in a gene panel test; generating an evaluation result for a plurality of evaluation items based on a first criterion for evaluating each of the evaluation items and at least the information on the genetic mutation; determining the level of pathogenicity based on a second criterion for determining the level of pathogenicity and the evaluation result; determining an undetermined item for which the evaluation result is undetermined among the plurality of evaluation items, and determining whether the level of pathogenicity is tentative based on a result of the determination of the undetermined item, and generating the report that reports the level of pathogenicity according to a result of the determination on whether the level of pathogenicity is tentative.

## Description

### TECHNICAL FIELD

The disclosure relates to a report generating method of generating a report for reporting a level of pathogenicity of a genetic mutation. The invention also relates to a report generating device that generates a report for reporting a level of pathogenicity of a genetic mutation.

### BACKGROUND ART

A gene panel test that can comprehensively examine multiple genetic abnormalities using the next-generation sequencer (NGS) has emerged and is beginning to be used in the field of oncology and other fields as a technology that plays an important role in personalized medicine. Also, in the field of genetic diseases, which account for many intractable diseases, genetic analysis using NGS is being used to elucidate a causative gene and to research and develop a therapeutic drug, and an expectation for a gene panel test equipped with a candidate causative gene is increasing.

When a genetic mutation associated with a hereditary disease is detected in a test, it is recommended to determine a level of pathogenicity of a genetic mutation based on a prescribed guideline. The guidelines for determining the level of pathogenicity of a genetic mutation include the ACMG/AMP guidelines, for example. In the guidelines, (1) based on the criteria of multiple evaluation items (28 items) for pathogenicity, the evaluation items applicable to the target genetic mutation are determined, and (2) based on the applicable evaluation items, the level of pathogenicity of the genetic mutation is determined.

Non-patent literature 1 ("Evaluation of ACMG-Guideline-Based Variant Classification of Cancer Susceptibility and Non-Cancer-Associated Genes in Families Affected by Breast Cancer", American Journal of Human Genetics, 2016 May 5;98(5):801-817) discloses the results of a comparison of the determination of the level of pathogenicity based on the ACMG/AMP guidelines with the results of the determination of the level of pathogenicity based on existing databases after performing a whole exome sequencing using samples obtained from subjects at high risk for breast and ovarian cancer. In addition, in determining the level of pathogenicity based on the ACMG/AMP guidelines, a method is disclosed to automatically determine whether some of the multiple evaluation items for pathogenicity are applicable or not.

### SUMMARY

In a gene panel test, specialists such as physicians and genetic counselors comprehensively interpret the test results, including the results of determining the level of pathogenicity of each genetic mutation, identify the causative gene, and formulate a treatment strategy. Therefore, it is important to determine the level of pathogenicity appropriately according to the guidelines. However, among the multiple evaluation items used to determine the level of pathogenicity, there are items which is difficult to obtain the information necessary to discriminate between applicable and not applicable in a short period of time.

For example, some of the multiple evaluation items in the ACMG/AMP guidelines require information on a genetic mutation and medical history of a blood relative, but it may be difficult to obtain information on a genetic mutation and medical history of a blood relative in a short period of time. In addition, some of the multiple evaluation items require data from disease patient cohorts, but it may take a long time to obtain cohort data including a sufficient number of patients due to rare diseases, etc.

Therefore, there may be a case in which the level of pathogenicity of a genetic mutation is determined based solely on the confirmed evaluation items while there are evaluation items for which the determination of applicable/non-applicable status is not yet finalized. However, additional analysis or investigation, etc. afterwards may determine some or all of the undetermined evaluation items, and in such a case, the number of determined evaluation items may increase, which may change the result of the determination of the level of pathogenicity obtained earlier. However, Non-Patent Document 1 does not describe the possibility that the results of the determination of the level of pathogenicity may be changed later.

Therefore, the purpose of the present invention is to provide a report generating method and a report generating device that can provide appropriate information even when the determined level of pathogenicity may be changed later.

A method of generating a report of the invention is a report generating method of generating a report to report a level of pathogenicity of a genetic mutation, comprising obtaining information on a genetic mutation detected in a gene panel test; generating an evaluation result for a plurality of evaluation items based on a first criterion for evaluating each of the evaluation items and at least the information on the genetic mutation; determining the level of pathogenicity based on a second criterion for determining the level of pathogenicity and the evaluation result; determining an undetermined item for which the evaluation result is undetermined among the plurality of evaluation items, and determining whether the level of pathogenicity is tentative based on a result of the determination of the undetermined item, and generating the report that reports the level of pathogenicity according to a result of the determination on whether the level of pathogenicity is tentative.

Also, the report generating device of the invention is a report generating device for generating a report to report a level of pathogenicity of a genetic mutation, comprising: a controller; and an output device, wherein the controller is programmed to obtain information on a genetic mutation detected in a gene panel test; generate an evaluation result for a plurality of evaluation items based on a first criterion for evaluating each of the evaluation items and at least the information on the genetic mutation; determine the level of pathogenicity based on a second criterion for determining the level of pathogenicity and the evaluation result; determine an undetermined item for which the evaluation result is undetermined among the plurality of evaluation items, and determine whether the level of pathogenicity is tentative based on a result of the determination of the undetermined item; and generate the report that reports the level of pathogenicity according to a result of the determination on whether the level of pathogenicity is tentative, and wherein the output unit outputs the report.

The present invention can provide a report generating method and a report generating device that can provide appropriate information even when the determined level of pathogenicity may be changed later.

### BRIEF DESCRIPTION OF DRAWINGS

FIG. 1 is a schematic diagram illustrating a report generating system according to a first embodiment;
FIG. 2 is a diagram illustrating an example of a test request information input screen displayed on a display unit of a data transmitting device
FIG. 3 is a flowchart illustrating an example of a process procedure executed by each control unit of the data transmitting device, a second report generating device, and a first report generating device;
FIG. 4 is a flowchart illustrating an example of a process procedure when the control unit of the first report generating device determines a level of pathogenicity of 1 genetic mutation;
FIGS. 5A and 5B are diagrams illustrating contents of 28 evaluation items specified by the ACMG/AMP guidelines;
FIGS. 6A and 6B are diagrams illustrating gene region information table;
FIG. 7 is a diagram illustrating a level of pathogenicity;
FIG. 8 is a diagram illustrating the second criterion for determining the level of pathogenicity of a genetic mutation using 28 evaluation items specified by the ACMG/AMP guidelines;
FIG. 9 is a flowchart illustrating an example of a process procedure when the control unit of the first report generating device assigns the level of pathogenicity in step S46;
FIG. 10 is a diagram illustrating an example of a master table stored in a memory unit of the first report generating device at the time of a positive judgement is made in step S28;
FIGS. 11A, 11B, and 11C are diagrams illustrating a case in which the level of pathogenicity is changed as an evaluation result is finalized;
FIG. 12A is a diagram illustrating a first result report according to a first embodiment;
FIG. 12B is a diagram illustrating a second result report according to the first embodiment;
FIG. 13 is a diagram illustrating partial view of a second region of the first result report or the second result report of a variation 1;
FIG. 14 is a diagram illustrating partial view of the second region of the first result report or the second result report of a variation 2;
FIG. 15 is a diagram illustrating partial view of the second region of the first result report or the second result report of a variation 3;
FIG. 16A is a diagram illustrating a summary report for a variation 4, and FIG. 16B is a diagram illustrating the detailed report for the variation 4;
FIG. 17 is a diagram illustrating the summary report for a variation 5;
FIG. 18 is a diagram illustrating the detailed report of the variation 5;
FIG. 19 is a diagram illustrating the summary report for a variation 6;
FIG. 20 is a diagram illustrating an input screen for entering an evaluation result of an undetermined item, which is displayed on the display unit of the data transmitting device in step S3;
FIG. 21 is a diagram illustrating the input screen of a variation;
FIGS. 22A and 22B are diagrams illustrating rules for determining the level of pathogenicity employed in a second embodiment;
FIG. 23 is a flowchart corresponding to FIG. 9 according to a second embodiment;
FIG. 24 is a schematic diagram illustrating a report generating system 101 according to a third embodiment;
FIG. 25 is a flowchart illustrating an example of a process procedure executed by each control unit of the data transmitting device and report generating device according to a third embodiment;
FIG. 26 is a flowchart illustrating an example of a process procedure executed by each control unit of the data transmitting device and report generating device according to a fourth embodiment; and
FIG. 27 is a diagram illustrating an example of the test request information input screen displayed on the display unit of the data transmitting device according to a fourth embodiment.

### DETAILED DESCRIPTION

A report generating method and report generating system according to one or more embodiment is described with reference to the drawings. One or more embodiments described below are examples only, and the invention is not limited to the following embodiments. Also, in each of the following embodiments, the same symbol is attached to the same configuration in the drawings, and redundant explanations are omitted.

### First embodiment

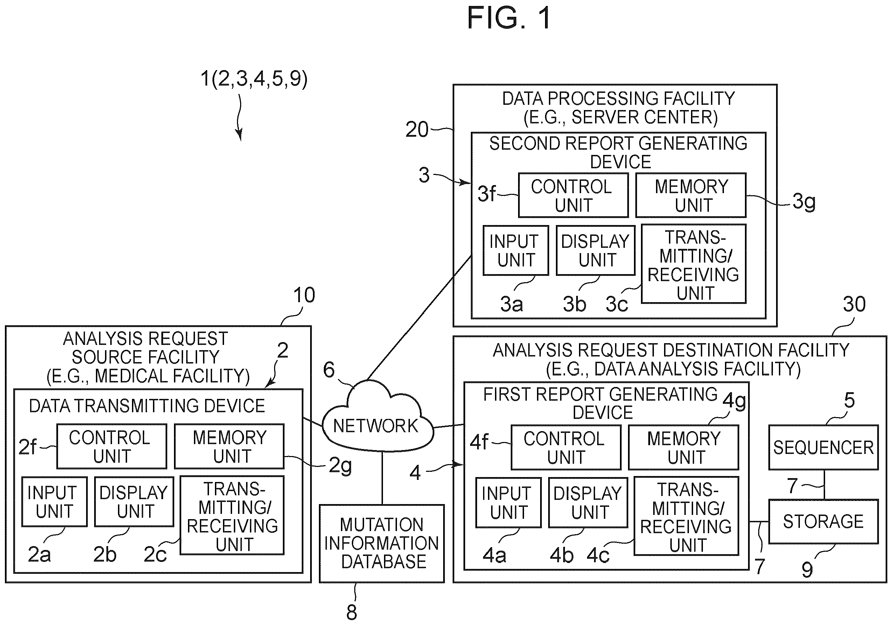

FIG. 1 is a schematic diagram of a report generating system 1 according to a first embodiment. As illustrated in FIG. 1, the report generating system 1 is equipped with a data transmitting device 2, a second report generating device 3, a first report generating device 4, a sequencer 5, and a storage (memory device) 9. The data transmitting device 2, the second report generating device 3, and the first report generating device 4 are connected to each other via a network 6, which is the internet. A mutation information database 8 is connected to the network 6. The first report generating device 4, the sequencer 5, and the storage 9 are connected to each other via a local area network 7. The first report generating device 4 is a device that generates evaluation results of each evaluation item specified in the ACMG/ AMP guidelines based on information on a genetic mutation, determines a level of pathogenicity based on the generated evaluation result, and generates a report for reporting the determined level of pathogenicity; and the second report generating device 3 is a device that determines a level of pathogenicity based on the evaluation results of each evaluation item generated by the first report generating device 4 and an additional evaluation result for an undetermined item and generates a report for reporting the determined level of pathogenicity.

The data transmitting device 2 is installed at an analysis request source facility 10, for example, a hospital (medical facility), a testing center, or a biomedical science laboratory. The data transmitting device 2 is a computer. The data transmitting device 2 has an input unit 2a, a display unit 2b, a transmitting/receiving unit 2c, a control unit 2f, and a memory unit 2g. The input unit 2a is used to input data and consists of a keyboard and a mouse. The display unit 2b consists of a liquid crystal panel and displays an image. The display unit 2b may consist of an organic EL panel. The input unit 2a and the display unit 2b may consist of a touch panel that integrates a touch sensor and a display. The transmitting/receiving unit 2c is an interface for transmitting and receiving data to and from an external device via the network 6 connected to the data transmitting device 2, for example, consists of an ethernet-compatible interface. The control unit 2f is a CPU, and the memory unit 2g consists of a solid-state drive (SSD) and a memory. Test request information for a gene panel test, which is input by a user of the data transmitting device 2 using the input unit 2a, is transmitted from the transmitting/receiving unit 2c to the second report generating device 3 via the network 6. The test request information is explained in detail later.

The second report generating device 3 is installed in a data processing facility 20, e.g., a server center. In the first embodiment, the data processing facility 20 is a different facility from the analysis request source facility 10, but the data processing facility 20 may be the same facility as the analysis request source facility 10. The data processing facility 20 may be a facility of a cloud service provider or a facility of a company that provides nucleic acid sequence analysis services. The second report generating device 3 may be a computer comprising a cloud system. The second report generating device 3 includes an input unit 3a, a display unit 3b, a transmitting/receiving unit 3c, a control unit 3f, and a memory unit 3g. The hardware configurations of the input unit 3a, the display unit 3b, the transmitting/receiving unit 3c, the control unit 3f, and the memory unit 3g are the same as those of the input unit 2a, the display unit 2b, the transmitting/receiving unit 2c, the control unit 2f, and the memory unit 2g, respectively. In the memory unit 3g, a first criterion for evaluating each of a plurality of evaluation items used to determine the level of pathogenicity and a second criterion for determining the level of pathogenicity based on the evaluation results of the plurality of evaluation items are stored. In addition, the first criterion and the second criterion may be obtained by the control unit 3f from the mutation information database 8. The second report generating device 3 sends the test request information received from the data transmitting device 2 from the transmitting/receiving unit 3c via the network 6 to the first report generating device 4. Also, the second report generating device 3 generates a second result report for reporting the level of pathogenicity based on an additional evaluation result received from the data transmitting device 2 and the information of the first result report for reporting the level of pathogenicity received from the first report generating device 4 and sends the second result report from the transmitting/receiving unit 3c to the data transmitting device 2 via the network 6. The first and second criteria, the additional evaluation result, the first result report, and the second report are described in detail later.

The first report generating device 4 is installed at an analysis request destination facility 30, e.g., a data analysis facility. In the first embodiment, the analysis request destination facility 30 is a different facility from both the analysis request source facility 10 and the data processing facility 20, but the analysis request destination facility 30 may be the same facility as the analysis request source facility 10 or the same facility as the data processing facility 20. The first report generating device 4 may be a computer comprising a cloud system. The first report generating device 4 can access to the mutation information database 8.

The first report generating device 4 has an input unit 4a, a display unit 4b, a transmitting/receiving unit 4c, a control unit 4f, and a memory unit 4g. The hardware configurations of the input unit 4a, the display unit 4b, the transmitting/receiving unit 4c, the control unit 4f, and the memory unit 4g are the same as those of the input unit 2a, the display unit 2b, the transmitting/receiving unit 2c, the control unit 2f, and the memory unit 2g, respectively. Similar to the memory unit 3g, in the memory unit 4g, a first criterion for evaluating each of the multiple evaluation items used to determine the level of pathogenicity and a second criterion for determining the level of pathogenicity based on the evaluation results of the multiple evaluation items are stored. In addition, the first criterion and the second criterion may be obtained by the control unit 4f from the mutation information database 8. Also, the first report generating device 4 generates the first result report based on the nucleic acid sequence data read from the storage 9, the reference sequence data obtained from the mutation information database 8, and the first and second criteria.

The sequencer 5 is a next-generation sequencer (NGS). Hereafter, when referring to a sequencer, it shall refer to a next-generation sequencer. The sequencer 5 is a device that reads base sequence information of nucleic acids, for example, the MiSeq system (manufactured by Illumina, Inc.), the NextSeq550 system (manufactured by Illumina, Inc.), the Ion GeneStudio S5 System (manufactured by Thermo Fisher Scientific Inc.), or the Ion Torrent Genexus System (manufactured by Thermo Fisher Scientific, Inc.), and other systems can be used. The sequencer 5 can read nucleic acid sequences from multiple library samples in a single sequence run. The storage 9 is a Network Attached Storage (NAS). The NAS is configured as a storage device that can be directly connected to a network.

In reading a nucleic acid sequence by the sequencer 5, the sequencer 5 accepts a sequence run ID, a specimen ID, and an index ID. The sequence run ID is identification information of the sequence run data. The sequencer 5 reads nucleic acid sequences of multiple library samples in one sequence run, i.e., one cartridge, and generates multiple nucleic acid sequence data corresponding to each library sample.

The library sample is a sample prepared for reading a nucleic acid sequence and is also called a library. Multiple library samples are prepared, for example, by pretreating each of the multiple specimens prepared from respective multiple subjects with a reagent and adding different index sequences to the multiple samples from each other. The library sample can be prepared using, for example, Onco Guide (registered trademark) NCC Onco Panel kit (manufactured by Sysmex Corporation). The specimen ID is information that identifies each library sample. The index ID is information that identifies index sequences attached to each library sample.

A user of the sequencer 5 dispenses a plurality of pre-prepared library samples into a single cartridge, sets the cartridge in the sequencer 5, and gives an instruction to start sequence reading. When the user instructs the sequencer 5 to start sequence reading, the sequencer 5 reads nucleic acid sequences for each of the multiple library samples, generates sequence run data, and records the sequence run data in the storage 9. The sequence run data includes, for each library sample, a specimen ID, an index ID, and a nucleic acid sequence data to which an index sequence is appended.

The mutation information database 8 consists, for example, of an external public sequence information database or a public known mutation information database. The control unit 4f of the first report generating device 4 checks each of the nucleic acid sequence data contained in the sequence run data read from the storage 9 with the reference sequence data stored in the mutation information database 8 and generates mutation information of genes for each of the nucleic acid sequence data.

The mutation information database 8 is an external public sequence information database, a publicly known mutation information database, etc. The public sequence information database includes the NCBI RefSeq (webpage, www.ncbi.nlm.nih.gov/refseq/), NCBI GenBank (webpage, www.ncbi.nlm.nih.gov/genbank/), UCSC Genome Browser, etc. Also, the publicly known mutation information database includes the COSMIC database (webpage, www.sanger.ac.uk/genetics/CGP/cosmic/), ClinVar database (webpage, www.ncbi.nlm.nih.gov/clinvar/), dbSNP (webpage, www.ncbi.nlm.nih.gov/SNP/), etc. In addition, the mutation information database 8 may be a publicly known mutation information database that includes frequency information for each race or animal species with respect to publicly known mutations. The publicly known mutation information database with such information includes HapMap Genome Browser release #28, Human Genetic Variation Browser (webpage, www.genome.med.kyoto-u.ac.jp/SnpDB/index.html) and 1000 Genomes (webpage, www.1000genomes.org/). Furthermore, the mutation information database 8 may also be any other public and/or commercial database and may be public and/or commercial databases, such as UniProt, HGMD, etc.

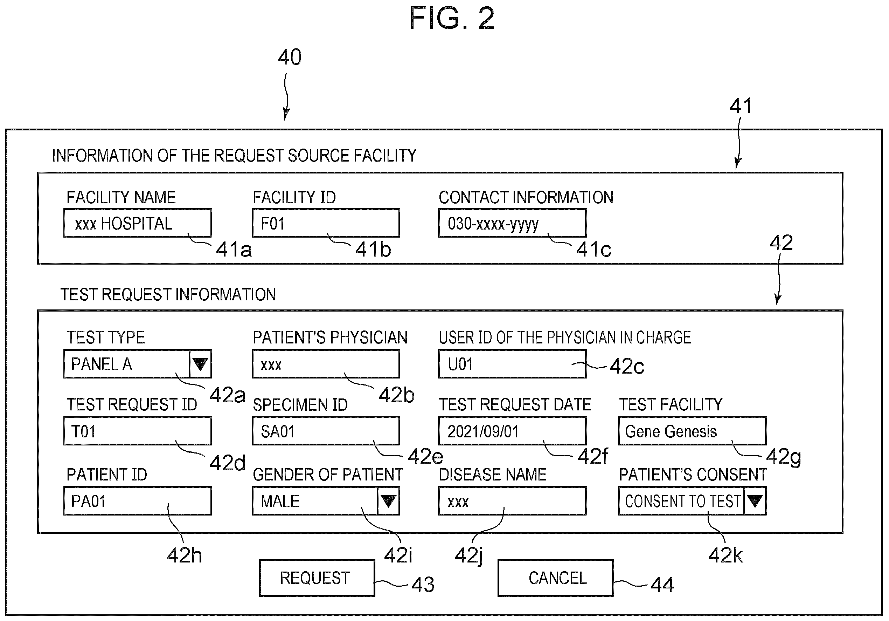

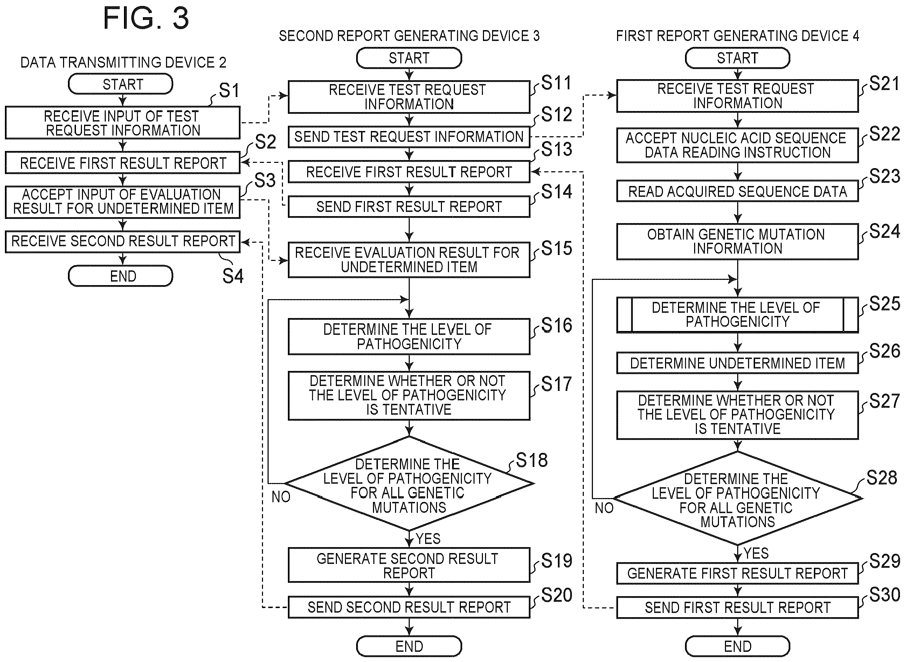

Next, a report generating method by the report generating system 1 is described. FIG. 3 is a flowchart illustrating processes performed by each of the control units 2f, 3f, and 4f of the data transmitting device 2, the second report generating device 3, and the first report generating device 4. Referring to FIG. 3, in step S1, the control unit 2f of the data transmitting device 2 displays a test request information input screen 40 (see FIG. 2) on the display unit 2b, accepts the input of test request information, and transmits the accepted test request information to the second report generating device 3.

FIG. 2 is a diagram illustrating an example of the test request information input screen 40 displayed on the display unit 2b in step S1. As illustrated in FIG. 2, the test request information input screen 40 displays a registration unit 41 of request source facility information, a registration unit 42 of test request information, a request operation unit 43, and a cancellation operation unit 44. The registration unit 41 of the request source facility information includes an input area 41a for entering a facility name, a display area 41b of a facility ID, and an input area 41c for inputting contact information. The input area 41a and the input area 41c are configured for a user to operate the input unit 2a of the data transmitting device 2 to input numerical values, letters, or symbols. The input area 41a is an area for inputting a facility name of the request source facility, and for example, xxx hospital is entered in the input area 41a.

In the display area 41b, a facility ID corresponding to a facility entered in the input area 41a is automatically displayed. This display is performed by the control unit 2f searching for a facility ID corresponding to the facility name. The memory unit 2g stores a facility table that shows the correspondence relationship between the facility ID and the facility name corresponding to the facility ID. By the control unit 2f referring to the facility table, the facility ID corresponding to the entered facility name is displayed in the display area 41b. The display area 41b shows F01, for example. When there is no facility ID corresponding to the entered facility name, an automatically generated facility ID is displayed. In addition, when there is no facility ID corresponding to the entered facility name, information indicating that the corresponding facility ID does not exist may be displayed on the test request information input screen 40.

The input area 41c is an area for inputting the representative contact information of a request source facility, and the representative telephone number of the request source facility is input. Also, the input area 41c may be made to input e-mail addresses, addresses, etc.

The registration unit 42 for the test request information includes an input area 42a for inputting a test type, an input area 42b for inputting a physician in charge, a display area 42c for displaying a user ID of the physician in charge, a display area 42d for displaying a test request ID, a display area 42e for displaying a specimen ID, a display area 42f for displaying the test request date, an input area 42g for inputting a test facility, a display area 42h for displaying a patient ID, an input area 42i for inputting a gender of the patient (subject), an input area 42j for entering the name of the disease, and an input area 42k for entering whether the patient consents or not. The input area 42a, the input area 42b, the input area 42g, the input area 42i, the input area 42j, and the input area 42k are configured so that a user operates the input unit 2a of the data transmitting device 2 to input numerical values, letters, or symbols.

The input area 42a is an area for inputting a panel test name used for a gene panel test. The input area 42a is configured in a pull-down list format, in which panel test types allowed by the report generating system 1 are registered, and the user selects a panel test type from the pull-down list by operating the input unit 2a. The input area 42b is an area for entering the name of a physician in charge of a patient. The display area 42c displays a user ID corresponding to the name information of the physician in charge. This display is performed by the control unit 2f searching for the user ID corresponding to the physician in charge. The storage unit 2g stores a facility table that shows the correspondence relationship between the user ID of the physician in charge and the name of the physician in charge corresponding to the user ID. By the control unit 2f referring to the facility table, the user ID corresponding to the inputted name of the physician in charge is displayed in the display area 42c. Also, when there is no user ID corresponding to the entered name, an automatically generated user ID may be displayed. Or, when there is no user ID corresponding to the entered name, information meaning that the corresponding user ID does not exist may be displayed on the test request information input screen 40.

In the display area 42d, a test request ID, which is an ID to identify individual test, is automatically displayed. Instead of the display area 42d, an input area for entering a test request ID individually assigned by a medical facility of the request source may be provided. In the display area 42e, a specimen ID, which is an ID to identify individual specimen, is automatically displayed. Instead of the display area 42e, an area for inputting the test ID individually assigned by the medical facility of the request source may be provided. In the display area 42f, the date when the test request is made is automatically registered.

The input area 42g is an area for entering a facility where a test of the genetic information is to be performed. In the display area 42h, a patient ID is automatically displayed. Instead of the display area 42h, an input area for entering the patient ID individually assigned by the medical facility of the request source may be provided. The input area 42i is an area for entering the patient's gender, and the input area 42j is an area for entering the patient's disease name. The input area 42k is an area for entering the patient's consent or lack thereof. The patient consent includes consent for testing, consent for providing anonymized test results to a third-party organization, and a combination of these. The content of patient consent is not limited to these. The "patient consent" information may be presented as a pull-down menu or radio button selection, or any combination from a list.

The request operation unit 43 is operated, for example, by placing the cursor on the image of the request operation unit 43 and clicking or double-clicking it with the mouse. When the request operation unit 43 is operated, the information displayed in the input areas/display areas 41a to 41c and the input areas/display areas 42a to 42k is sent to the second report generating device 3 as test request information. The cancel operation unit 44 is operated, for example, by placing the cursor on the image of the request operation unit 43 and clicking or double-clicking it with the mouse. When the cancel operation unit 44 is operated, the information that has been entered and displayed on the test request information input screen 40 up to that point is deleted. Also, the test request information needs to only include the information necessary to request a test, and some of the information displayed in the input areas/display areas 41a to 41c and the input areas/display areas 42a to 42k may be omitted.

Referring again to FIG. 3, in step S11, the control unit 3f of the second report generating device 3 receives the test request information received from the data transmitting device 2, i.e., each of the information displayed in the input areas/display areas 41a to 41c and the input areas 42a to 42k/display areas of the test request information input screen 40 and stores the information in the memory unit 3g. In step S12, the control unit 3f sends the test request information to the first report generating device 4. Next, in step S21, the control unit 4f of the first report generating device 4 receives the test request information sent from the second report generating device 3, stores it in the storage unit 4g, and indicates on the display unit 4b that the test request information is accepted.

When a user of the first report generating device 4 or a user of the sequencer 5 recognizes that the test request information is accepted by the information displayed on the display 4b, the user prepares a library sample from the specimen identified by the specimen ID displayed in the input area 42e of the test request information input screen 40 and has the sequencer 5 read the nucleic acid sequence data. The sequencer 5 stores the sequence run data including the read nucleic acid sequence data in the storage 9. When the reading of the nucleic acid sequence data by the sequencer 5 is completed, the user of the first report generating device 4 operates the input unit 4a of the first report generating device 4 to instruct the reading of the nucleic acid sequence data. With this instruction, the control unit 4f accepts the instruction to read the nucleic acid sequence data by the user of the first report generating device 4 in step S22.

In step S23, the control unit 4f reads the nucleic acid sequence data (hereinafter referred to as "acquired sequence data") identified by the specimen ID displayed in the input area 42e of the test request information input screen 40 from the sequence run data stored in the storage 9. In step S24, the control unit 4f detects all genetic mutations in the acquired sequence data based on the acquired sequence data and the reference sequence data obtained from the mutation information database 8 and obtains the information of the genetic mutation. The detection of a genetic mutation can be performed by the following known methods. First, the position on the reference sequence where the degree of consistency between the acquired sequence data and the reference sequence data meets a predetermined criterion is identified, and the bases corresponding to each other in the acquired sequence data and the reference sequence data are compared. When there is no discrepancy between the corresponding bases, it is determined that there is no genetic mutation. On the other hand, when there is a discrepancy between the corresponding bases, the discrepant base is detected as a genetic mutation. In step S24, the control unit 4f also determines the genetic form, mutant allele frequency, homo or hetero, mutation location (base), and mutation location (amino acid) based on the acquired sequence data and the reference sequence data obtained from the mutation information database 8.

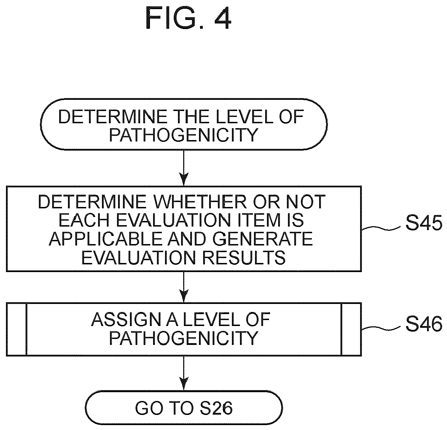

In step S25, the control unit 4f determines a level of pathogenicity for one of the genetic mutations detected in step S24. The details of the process of determining the level of pathogenicity are described with reference to FIG. 4. In step S45, the control unit 4f determines whether each of the 28 evaluation items specified by the ACMG/AMP guidelines (Richards et al., Genetics in Medicine volume 17, pages 405-423(2015)) is applicable based on the first criterion stored in the memory unit 4g and one of the genetic mutations detected in step S24 and generates an evaluation result.

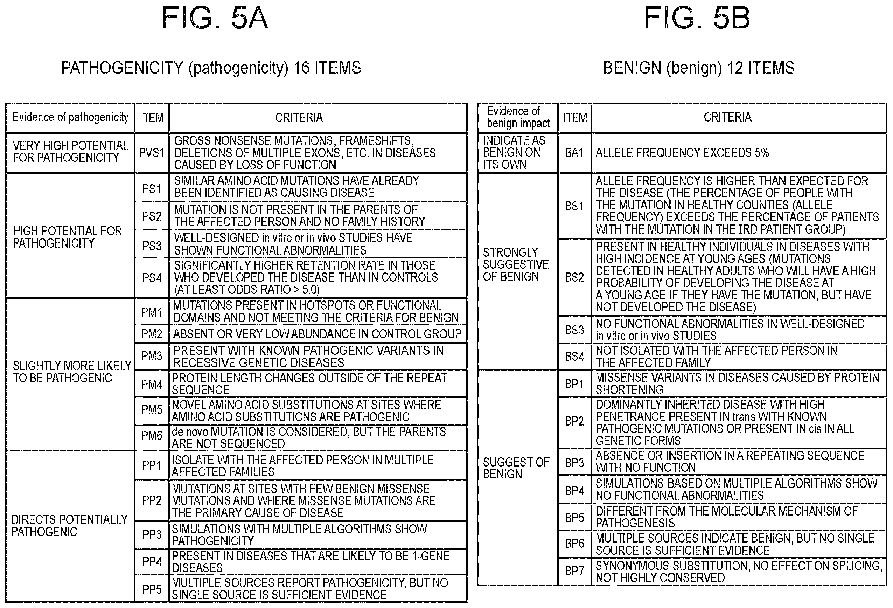

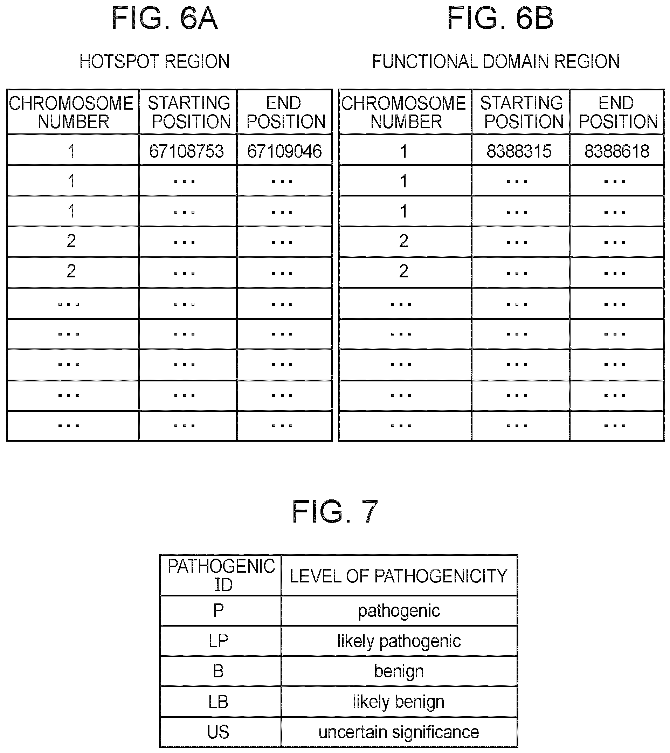

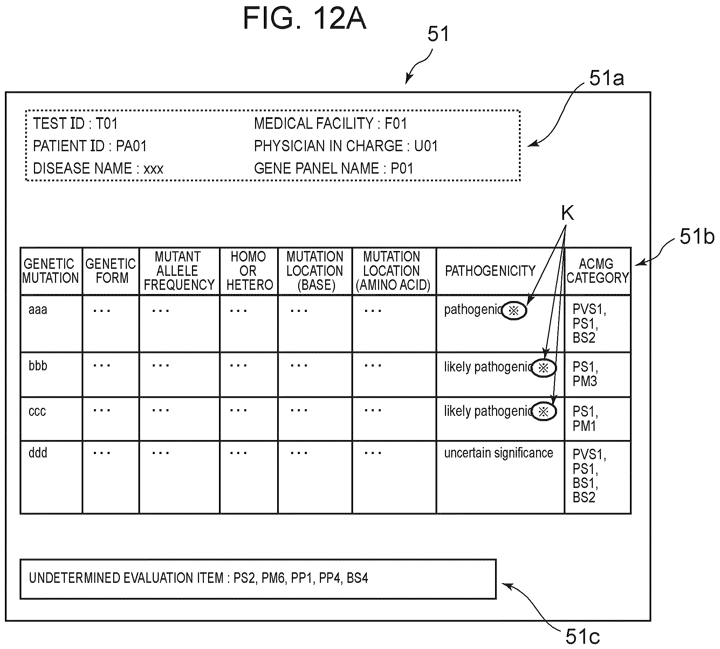

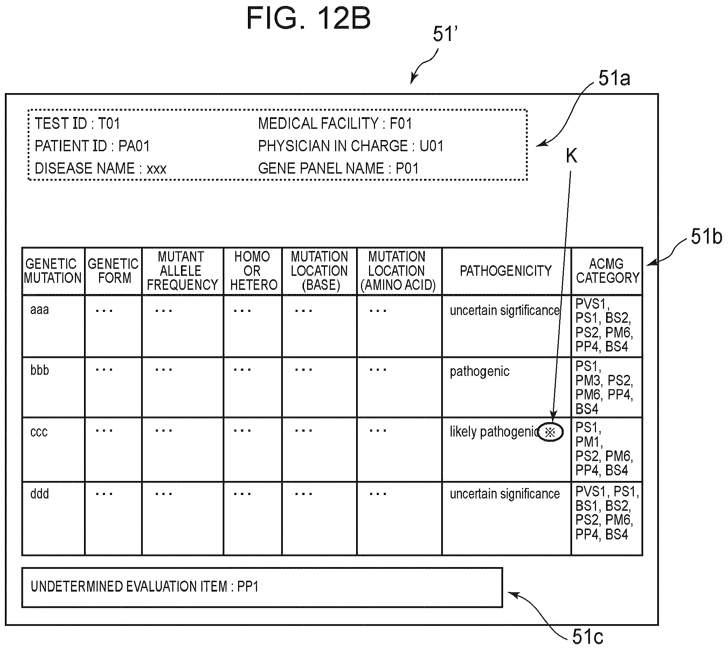

The process of step S45 is described in detail below with reference to FIGS. 5A, 5B and FIGS. 6A and 6B. FIGS. 5A and 5B are diagrams illustrating the contents of 28 evaluation items specified by the ACMG/AMP guidelines. As illustrated in FIG. 5A and 5B, the evaluation items include 16 items related to pathogenicity illustrated in FIG. 5A and 12 items related to benignity illustrated in FIG. 5B. Next, some of the evaluation items are described in detail.

PVS1, which is included in the 16 items for pathogenicity, is an evaluation item that, if applicable, suggests a very high likelihood of being pathogenic. PVS1 can be determined applicable or not by determining the presence or absence of gross nonsense mutations, frameshifts, deletions of multiple exons, etc., in diseases caused by loss-of-function. In the memory unit 4g of the first report generating device 4 and the memory unit 3g of the second report generating device 3, the nucleic acid sequence data with a genetic mutation that is determined to correspond to PVS1 based on the ACMG/AMP guidelines is stored as the first criterion. In step S45 (FIG. 4), the control unit 4f refers to the nucleic acid sequence data having a genetic mutation stored in the storage unit 4g and determines whether one of the genetic mutations detected in step S24 is identical to the genetic mutation that the nucleic acid sequence data stored in the storage unit 4g has; if it is identical, it is determined to correspond to PVS1; if it is not identical, it is determined not to correspond to PVS1.

PS1 is an evaluation item that, if applicable, suggests a high likelihood of being pathogenic. PS1 can be determined applicable or not by determining whether a similar amino acid mutation has already been identified as causing the disease. In the memory unit 4g of the first report generating device 4 and the memory unit 3g of the second report generating device 3, the nucleic acid sequence data with a genetic mutation that is determined to correspond to PS1 based on the ACMG/AMP guidelines is stored as the first criterion. In step S45 (FIG. 4), the control unit 4f refers to the nucleic acid sequence data having a genetic mutation stored in the storage unit 4g and determines whether one of the genetic mutations detected in step S24 is identical to the genetic mutation that the nucleic acid sequence data stored in the storage unit 4g has; if it is identical, it is determined to correspond to PS1; if it is not identical, it is determined not to correspond to PS1.

PM1 is an evaluation item that, if applicable, is determined to have a slightly higher likelihood of being pathogenic. PM1 can be determined applicable or not by determining whether the mutation is present in a hotspot or functional domain region and does not meet the criteria for benign.

FIGS. 6A and 6B diagrams illustrating gene region information tables and diagrams explaining about a hotspot region (FIG. 6A) and a functional domain region (FIG. 6B). In the hotspot region table illustrated in FIG. 6A, three hotspots exist for each gene on chromosome 1; for example, a first hotspot is a nucleic acid sequence portion from base 67108753 to base 67109046 on chromosome 1. Each hotspot region is stored in the memory unit 4g and the memory unit 3g as the first criterion. In step S45 (FIG. 4), the control unit 4f determines whether one of the genetic mutations detected in step S24 exists in the hotspot region stored in the memory unit 4g, and if it exists, determines that it corresponds to PM1, and if not, determines that it does not correspond to PM1.

In the functional domain region table illustrated in FIG. 6B, three functional domain regions exist in the gene on chromosome 1; for example, a first functional domain region is a nucleic acid sequence portion from base 8388315 to base 8388618 on chromosome 1. Each functional domain region is stored in the memory unit 4g and the memory unit 3g as the first criterion. In step S45 (FIG. 4), the control unit 4f determines whether one of the genetic mutations detected in step S24 exists in the functional domain region stored in the memory unit 4g, and if it exists, determines that it corresponds to PM1, and if not, determines that it does not correspond to PM1.

The first criterion is not limited to the above-mentioned examples and includes, for example, at least one of the following (1) to (7).
(1) name of the disease,
(2) information on at least one of the type of a genetic mutation, the location, and the amino acid and/or base altered by a mutation,
(3) information including at least one of the genetic information and medical history of a blood relative,
(4) information on the relationship between a disease and a genetic mutation,
(5) frequency of a genetic mutation in a control population and/or disease population,
(6) region information for each gene,
(7) information obtained by a prediction algorithm.

The "type of mutation" includes a nonsense mutation, frameshift mutation, deletion of multiple exons, missense mutation, etc. Also, the "location of mutation" includes the number of bases from the beginning of the genome or the transcript of the gene, etc. In addition, the "information on the relationship between a disease and a genetic mutation" includes, for example, the level of pathogenicity of the known genetic mutation in the disease, its rationale, information on the amino acid substitution or genetic form of the genetic mutation that is identified as pathogenic (pathogenic, likely pathogenic) in the disease, etc.

The "gene region information" includes, for example, genetic mutation information on the hotspot region, the functional domain region, a repetitive regions, etc.

The "information obtained by the prediction algorithm" is information on the interpretation of mutations obtained by a computer simulation calculation, etc., and includes, for example, a predicted value of the effect of a mutation on a protein function by SIFT. The information can be obtained using the prediction algorithm in a table 2 of the following document. S. Richards et al., "Standards and guidelines for the interpretation of sequence variants: a joint consensus recommendation of the American College of Medical Genetics and Genomics and the Association for Molecular Pathology," Genetics in Medicine 2015 17:5, vol. 17, no. 5, pp. 405-423, Mar. 2015, doi: 10.1038/gim.2015.30.

Referring again to FIG. 4, in step S46, the control unit 4f assigns a level of pathogenicity with respect to one of the genetic mutations detected in step S24, based on the second criterion for determining the level of pathogenicity and the result (evaluation result) of whether each evaluation item is applicable in step S45.

FIG. 7 is a diagram illustrating the level of pathogenicity. As illustrated in FIG. 7, the level of pathogenicity is indicated on a 5-point scale. A pathogenicity ID of P for "pathogenic" is the highest level of pathogenicity and is information indicating the presence of pathogenicity. Also, the pathogenicity ID of LP for "likely pathogenic" is the second highest level of pathogenicity and is information indicating possible pathogenic potential. In addition, the pathogenicity ID of B for "benign" is the lowest level of pathogenicity and is information indicating that the disease is benign. Furthermore, the pathogenicity ID of LB for "likely benign" is the second lowest level of pathogenicity and is information indicating the disease is likely to be benign. The pathogenicity ID of US for "uncertain significance" is information indicating that the presence or absence of pathogenicity is unknown, and the level of pathogenicity is lower than "likely pathogenic" and higher than "likely benign".

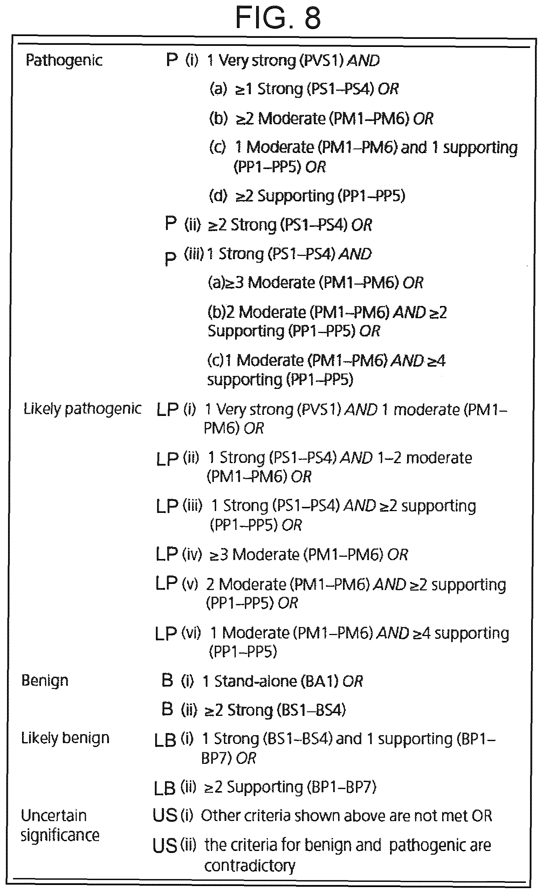

FIG. 8 is a diagram illustrating the second criterion for determining the level of pathogenicity. The second criterion is set according to the ACMG/AMP guidelines. For example, when at least one of P(i), P(ii), and P(iii), which are the criteria for "pathogenic," is met, it is determined to be "pathogenic". The criterion P(i) indicates that the evaluation result of step S45 corresponds to PVS 1 and at least one of criteria P(i)(a) to P(i)(d) is met, it is determined to be "pathogenic". P(i)(a) is satisfied when the evaluation result of step S45 corresponds to at least one of PS1-PS4, P(i)(b) is satisfied when the evaluation result of step S45 corresponds to two or more of PM1-PM6, P(i)(c) is satisfied when the evaluation result of step S45 corresponds to one of PM1-PM6 and one of PP1-PP5, and P(d) is satisfied when the evaluation result of step S45 corresponds to two or more of PP1-PP5.

The criterion P(ii) indicates when the evaluation result of step S45 corresponds to two or more of PS1-PS4, it is determined to be "pathogenic". The criterion P(iii) indicates when the evaluation result of step S45 corresponds to at least one of PS1-PS4 and at least one of the criteria P(iii)(a) to P(iii)(c) is met, it is determined to be "pathogenic". P(iii)(a) is satisfied when the evaluation result of step S45 corresponds to three or more of PM1-PM6, P(iii)(b) is satisfied when the evaluation result of step S45 corresponds to two of PM1-PM6 and two or more of PP1-PP5, and P(iii)(c) is satisfied when the evaluation result of step S45 corresponds to one of PM1-PM6 and four or more of PP1-PP5.

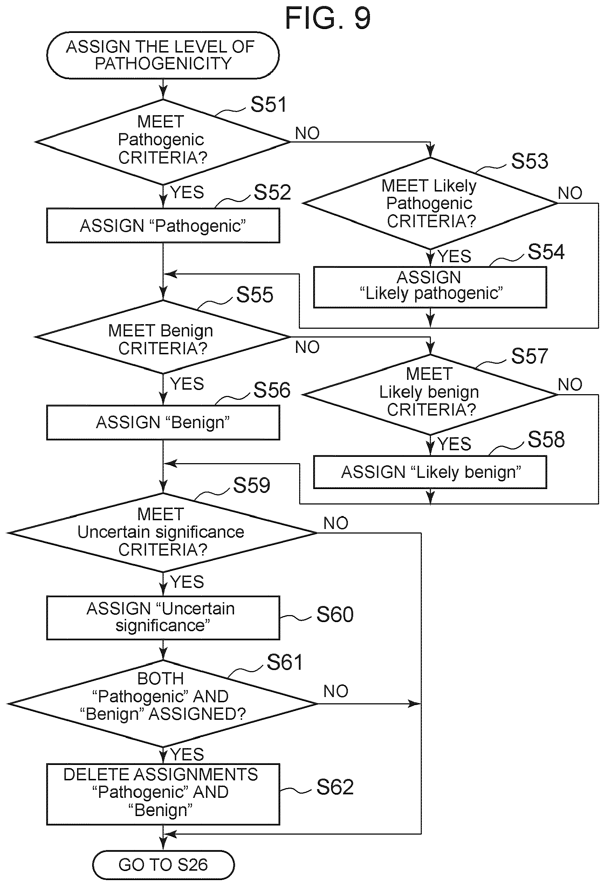

Referring again to FIG. 4, in step S46, the control unit 4f assigns either "pathogenic", "likely pathogenic", "benign", "likely benign", or "uncertain significance" to one of the genetic mutations detected in step S24, based on the second criterion and the evaluation result of step S45. This process is detailed below. FIG. 9 is a flowchart illustrating an example of a process procedure in which the control unit 4f of the first report generating device 4 assigns a level of pathogenicity in step S46. Referring to FIG. 9, in step S51, the control unit 4f determines whether one of the genetic mutations detected in step S24 meets the criteria for pathogenic based on the second criteria P(i) to P(iii) corresponding to pathogenic illustrated in FIG. 8. When a positive decision is made in step S51, the genetic mutation is assigned as pathogenic, and the process moves on to step S55.

On the other hand, when a negative decision is made in step S51, the process moves on to step S53, and the control unit 4f determines whether the genetic mutation meets the criteria for likely pathogenic based on the second criteria LP(i)-LP(vi) corresponding to likely pathogenic illustrated in FIG. 8. When a positive decision is made in step S53, the genetic mutation is assigned as likely pathogenic, and the process moves to step S55. On the other hand, when a negative decision is made in step S53, the control unit 4f moves the process to step S55.

In step S55, the control unit 4f determines whether the genetic mutation meets the criteria for benign based on the second criteria B(i)-B(ii) corresponding to benign illustrated in FIG. 8. When a positive decision is made in step S55, the control unit 4f assigns benign to the genetic mutation in step S56 and moves the process to step S59.

On the other hand, when a negative decision is made in step S55, the process moves to step S57, and the control unit 4f determines whether the genetic mutation meets the criteria for likely benign based on the second criteria LB(i)-LB(ii) corresponding to likely benign illustrated in FIG. 8. When a positive decision is made in step S57, the control unit 4f assigns likely benign to the genetic mutation in step S58 and moves the process to step S59. On the other hand, when a negative decision is made in step S57, the control unit 4f moves the process to step S59.

In step S59, the control unit 4f determines whether the genetic mutation meets the second criteria US(i) to US(ii) corresponding to uncertain significance illustrated in FIG. 8. Specifically, the control unit 4f determines that the criterion of uncertain significance is met when none of the criteria of pathogenic, likely pathogenic, benign, and likely benign is met. Also, in rare cases, a case that both pathogenic and benign are assigned occurs. In this case, the control unit 4f also determines that the uncertain significance criterion is met.

When a positive decision is made in step S59, in step S60, the control unit 4f assigns uncertain significance to the genetic mutation and moves the process to step S61. On the other hand, when a negative decision is made in step S59, the control unit 4f returns the process to step S26 (FIG. 3). In step S61, the control unit 4f determines if both pathogenic and benign are assigned or not. When a positive decision is made in step S61, in step S62, the control unit 4f deletes pathogenic and benign and returns the process to step S26 (FIG. 3). Also, when a negative decision is made in step S61, the control unit 4f does not execute step S 62 and returns the process to step S26 (FIG. 3).

The level of pathogenicity assigned in step S46 (FIG. 4, FIG. 9) may change with additional evaluations later. This is explained below. The first criterion includes criteria other than the genetic mutation of the subject, such as criteria related to genetic mutations of the subject's blood relatives and criteria related to medical histories of the subject's blood relatives. Therefore, in determining whether each evaluation item in step S45 (FIG. 4) is applicable, there may be an evaluation item that cannot be determined solely from the information on the genetic mutation of the subject detected in step S24, i.e., an evaluation item for which the evaluation result is not yet determined. In such cases, the level of pathogenicity assigned in step S46 may be subject to change later and is tentative information.

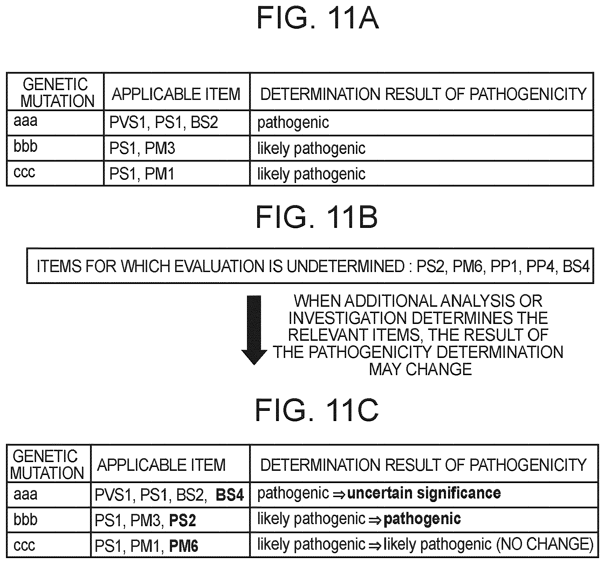

FIGS. 11A, 11B, and 11C is a diagram illustrating an example in which an evaluation result of an evaluation item (applicable or not) is later added or changed and confirmed, resulting in a change in the level of pathogenicity. In the example illustrated in FIG. 11A, for a genetic mutation "aaa", the evaluation result of step S45 corresponds to evaluation items PVS1, PS1, and BS2, and as a result, the level of pathogenicity is determined to be pathogenic. Also, for a genetic mutation "bbb", the evaluation result of step S45 corresponds to evaluation items PS1 and PM3, and as a result, the level of pathogenicity is determined to be likely pathogenic. Furthermore, for a genetic mutation "ccc", the evaluation result of step S45 corresponds to evaluation items PS1 and PM1, and as a result, the level of pathogenicity is determined to be likely pathogenic.

In addition, in this example, as illustrated in FIG. 11B, the determination result of pathogenicity in FIG. 11A is the result of evaluation items PS2, PM6, PP1, PP4, and BS4 being determined as yet undetermined because there was insufficient information to determine whether the evaluation items PS2, PM6, PP1, PP4, and BS4 are applicable in step S45. In this example, when information for determining whether the evaluation items PS2, PM6, PP 1, PP4, and BS4 are applicable is obtained by an additional analysis or an investigation, as illustrated in FIG. 11C, for example, a new evaluation item BS4 may be applicable for the genetic mutation "aaa". In this case, when the evaluation results of each evaluation item are applied to the second criterion, the level of pathogenicity of the genetic mutation "aaa" changes from pathogenic to uncertain significance. Similarly, for example, a new evaluation item may fall under PS2 for the genetic mutation "bbb". In this case, the level of pathogenicity of the mutation "bbb" changes from likely pathogenic to pathogenic. Also, in some cases, such as the case of the genetic mutation "ccc", the level of pathogenicity does not change even if a new evaluation item PM6 is applicable.

Thus, when there is an evaluation item for which the evaluation result is not yet finalized, the level of pathogenicity may be changed, and the pathogenicity level may be tentative rather than finalized. Therefore, in step S26 illustrated in FIG. 3, the control unit 4f determines an evaluation item that has not yet been determined (undetermined item) from each evaluation item evaluated in step S45. Specifically, the control unit 4f determines whether each of the first criteria corresponding to each evaluation item is a criterion related only to the information on the genetic mutation detected in step S24, or is a criterion related to other information. For example, the evaluation item PVS1 is a criterion that relates only to a genetic test result of the subject, and whether it is applicable can be determined by the information on the genetic mutation detected in step S24; therefore, the control unit 4f determines that the evaluation result of the evaluation item PVS1 is confirmed. On the other hand, the evaluation item PS2 is a criterion related to genetic mutations of the subject's parents, and whether or not it is applicable cannot be determined only by the information on the genetic mutation detected in step S24; therefore, the control unit 4f determines that the evaluation result of the evaluation item PS2 is not yet determined, i.e., the evaluation item PS2 is an undetermined item. The control unit performs the same determination for 28 evaluation items to determine the undetermined item.

Next, in step S27, the control unit 4f determines whether the level of pathogenicity determined in step S25 is tentative or not. Specifically, the control unit 4f determines whether the level of pathogenicity changes or not after changing the evaluation result of the undetermined item determined in step S26 from the evaluation result when the level of pathogenicity is assigned in step S46 and determining the level of pathogenicity again. When changing the evaluation result of at least one undetermined item results in a change in the level of pathogenicity, the control unit 4f determines that the level of pathogenicity is tentative. When changing the evaluation result of an undetermined item does not result in a change in the level of pathogenicity, the control unit 4f determines that the level of pathogenicity is not tentative. The control unit 4f also determines that the level of pathogenicity is not tentative even when it is determined that there is no undetermined item in step S26.

Next, in step S28, the control unit 4f determines whether the level of pathogenicity has been determined for all genetic mutations detected in step S24. When a positive determination is made in step S28, the control unit 4f proceeds to step S29. When a negative determination is made in step S28, the control unit 4f returns the process to step S25 and repeats processes of steps S25 to S27 until the level of pathogenicity is determined for all genetic mutations.

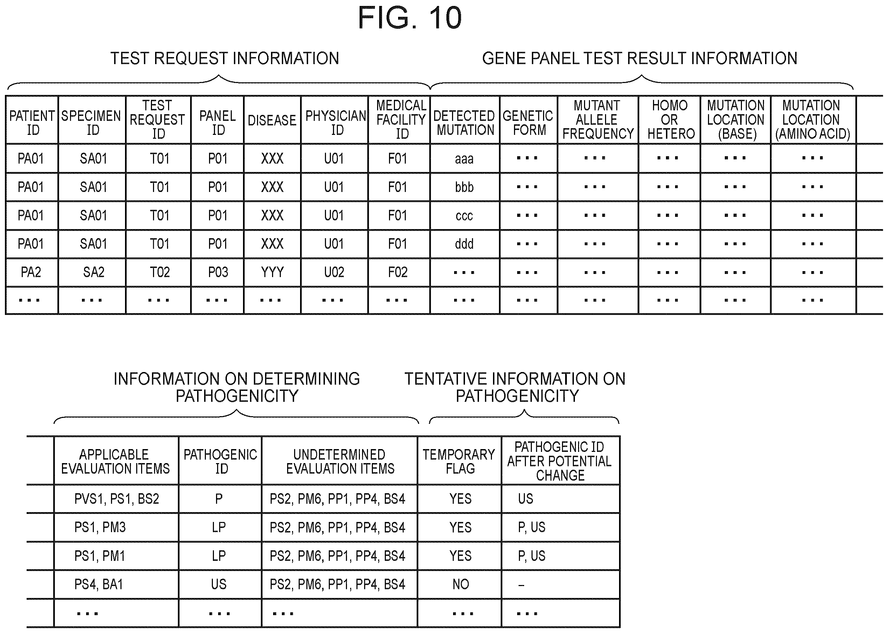

FIG. 10 is a diagram illustrating an example of a master table stored in the memory unit 4g at the time of the positive decision in step S28. As illustrated in FIG. 10, in the master table, the patient ID, specimen ID, test request ID, panel ID, disease, physician ID, medical facility ID, detected genetic mutation, genetic form, mutant allele frequency, homo or hetero, mutation location (base), mutation location (amino acid), applicable evaluation item, pathogenicity ID, undetermined evaluation item, tentative flag, and pathogenicity ID that may change are stored in a tied state.

The patient ID, specimen ID, test request ID, panel ID, disease, physician ID, and medical facility ID are information based on the test request information received in step S21. The detected genetic mutation, genetic form, mutant allele frequency, homo or hetero, mutation location (base), and mutation location (amino acid) are information based on the genetic mutation information obtained in step S24. In addition, the information on the applicable evaluation item, pathogenicity ID, and undetermined evaluation item is based on the information related to the evaluation item evaluated in step S45, the level of pathogenicity assigned in step S46, and the undetermined item determined in step S26. Specifically, the applicable evaluation item is the evaluation item determined to be applicable in step S45. The pathogenicity ID is the pathogenicity ID (FIG. 7) corresponding to the level of pathogenicity assigned in step S46. The undetermined evaluation item is the undetermined item determined in step S26. In addition, the tentative flag and the pathogenicity ID that may change are tentative information for the level of pathogenicity, and the tentative flag becomes "yes" when the level of pathogenicity is determined to be tentative in step S27 and "no" when the level of pathogenicity is determined not to be tentative. The pathogenicity ID that may change is a pathogenicity ID that indicates the level of pathogenicity after a change in the evaluation result of an undetermined item for a level of pathogenicity determined to be tentative.

For example, the data in the first row of the master table is: the patient ID is PA01, the specimen ID is SA01, the test request ID is T01, the panel ID is P01, the disease is XXX, the physician ID is U01, the medical facility ID is F01, the detected genetic mutation is aaa, the applicable evaluation items are PVS1, PS1, and BS2, the pathogenicity ID is P (pathogenic), the undetermined evaluation items are PS2, PM6, PP1, PP4, and BS4, the tentative flag is "yes", and the pathogenicity ID that may change is US (uncertain significance). Since the tentative flag is "yes" and the pathogenicity ID that may change is US, it indicates that the pathogenicity ID (P) for the genetic mutation aaa is tentative and may later change to US. In addition, in the example of the master table, the specimen IDs of the data in rows 1 to 4 are the same, and the detected mutations are different. Therefore, four genetic mutations (aaa, bbb, ccc, ddd) are detected in the specimen (library sample) with the specimen ID.

Referring again to FIG. 3, the control unit 4f generates a first result report in step S29.

FIG. 12A is a diagram illustrating an example of a first result report 51 of the first embodiment. The first result report 51 is a report generated based on the information of the same specimen in the master table illustrated in FIG. 10 (in the example in FIG. 10, the information in rows 1 through 4). As illustrated in FIG. 12A, the first result report 51 includes a first region 51a indicating bibliographic information, a second region 51b indicating the level of pathogenicity, and a third region 51c indicating an undetermined evaluation item (undetermined item). In the first region 51a, the test ID, medical facility, patient ID, physician in charge, patient name, and name of the gene panel are entered.

The second region 51b is described in a tabular format. In this table, each row lists the genetic mutation, genetic form, mutant allele frequency, homo or hetero, mutation location (base), mutation location (amino acid), pathogenicity, and ACMC category. The ACMC category is an evaluation item that is determined applicable and confirmed as applicable and is listed in the "Applicable Evaluation Item" column of the master table.

The pathogenicity is the level of pathogenicity determined, and the level corresponding to the pathogenicity ID listed in the "Pathogenicity ID" column of the master table is indicated by a letter. When the "Tentative Flag" in the master table is "Yes", a symbol "*" is added, as indicated by an arrow k. The symbol "*" means that the level of pathogenicity listed in the pathogenicity column may be subject to change and that the level of pathogenicity is tentative.

In the example illustrated in FIG. 12A, the symbol "*" is placed to the right of pathogenic, which is the level of pathogenicity corresponding to the genetic mutation "aaa", likely pathogenic, which is the level of pathogenicity corresponding to the genetic mutation "bbb", and likely pathogenic, which is the level of pathogenicity corresponding to the genetic mutation "ccc". Thus, the levels of pathogenicity of these three mutations are tentative information. On the other hand, the symbol "*" is not placed to the right of uncertain significance, which is the level of pathogenicity corresponding to the genetic mutation "ddd". Therefore, the level of pathogenicity of the genetic mutation "ccc" is determined to be uncertain significance.

In the third region 51c, the evaluation items that are not determined are described. In the example illustrated in FIG. 12A, all the evaluation items listed in rows 1 through 4 of "Undetermined Evaluation Item" in the master table are listed. Thus, in the example illustrated in FIG. 12A, PS2, PM6, PP1, PP4, and BS4 are listed in the third region 51c. Therefore, it is easy to understand that the evaluation items PS2, PM6, PP1, PP4, and BS4 are not used to determine the levels of pathogenicity of the genetic mutations "aaa", "bbb", "ccc", and "ddd" reported by the first result report 51. Referring again to FIG. 3, in step S30, the control unit 4f sends the first result report generated in step S29 to the second report generating device 3 as an electronic file via the transmitting/receiving unit 4c.

In step S13, the control unit 3f of the second report generating device 3 receives the first result report transmitted from the first report generating device 4. In step S14, the control unit 3f sends the first result report as an electronic file to the data transmitting device 2 via the transmitting/receiving unit 3c.

In step S2, the control unit 2f of the data transmitting device 2 receives the first result report from the second report generating device 3 and stores it in the memory unit 2g. This allows the physician in charge of the subject (patient) to display and view the first result report stored in the memory unit 2g on the display unit 2b at any time. In step S3, the control unit 2f accepts input of an evaluation result for an evaluation item that is shown to be undetermined in the first result report (an evaluation item listed in the third region 51c) and transmits the accepted evaluation result (an additional evaluation result) to the second report generating device 3 via the transmitting/receiving unit 2c.

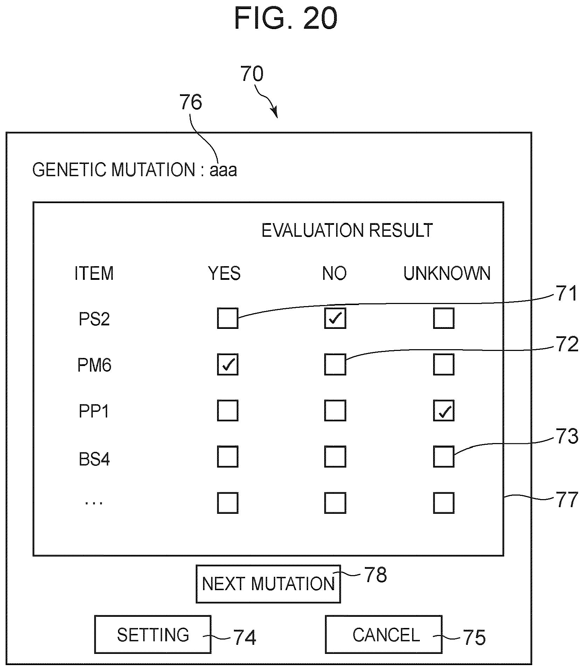

FIG. 20 is a diagram illustrating an input screen 70 for entering an evaluation result of an undetermined item, which is displayed on the display unit 2b of the data transmitting device 2 in step S3.

The input screen 70 includes a display area 76 for displaying a genetic mutation and an evaluation result input area 77 for inputting an evaluation result of an evaluation item that is shown to be undetermined in the first result report. The input screen 70 is displayed for each detected genetic mutation. The display area 76 is displayed one of the genetic mutations listed in the first result report (FIGS. 12A and 12B). The evaluation result input area 77 is displayed all of the undetermined evaluation items listed in the third region 51c of the first result report 51 (FIGS. 12A and 12B). Corresponding to each evaluation item, a check box 71 for entering a judgment result of "YES" indicating that the item is applicable, a check box 72 for entering a judgment result of "NO" indicating that the item is not applicable, and a check box 73 for entering "Unknown" indicating that it is unclear whether the item is applicable. The user of the data transmitting device 2 separately obtains the genetic information and medical history information of the subject's blood relatives, e.g., parents, determines whether each evaluation item listed in the evaluation result input area 77 is applicable based on the information obtained, and checks one of the check boxes 71, 72, and 73 using a mouse. In the example illustrated in FIG. 20, for the evaluation item PS2, the check box 72 indicating "NO" is checked because the user determines that the item does not fall under PS2, even considering the information obtained separately by the user, and for the evaluation item PM6, the check box 71 indicating "YES" is checked because the user determines that the item falls under PM6, considering the information obtained separately by the user.

The input screen 70 further includes a display operation unit 78, a setting operation unit 74, and a cancel operation unit 75. The display operation unit 78 is operated, for example, by placing the cursor on the image of the display operation unit 78 and clicking or double-clicking it with the mouse. When the user finishes checking all evaluation items and operates the display operation unit 78, the genetic mutation displayed in the display area 76 is changed to another genetic mutation, the checks entered in the check boxes 71, 72, and 73 are cleared, and the screen is switched to the screen for entering an evaluation result for the other genetic mutation. The display operation unit 78 is deactivated when the input of evaluation results for all detected genetic mutations is completed. The setting operation unit 74 is deactivated in the initial screen but is activated when the display operation unit 78 is deactivated. The setting operation unit 74 is operated, for example, by placing the cursor over the image of the setting operation unit 74 and clicking or double-clicking it with the mouse. When the user completes checking on the evaluation items for all genetic mutations and operates the setting operation unit 74, the control unit 2f sends the information on whether each evaluation item is applicable or unknown to the second report generating device 3 via the transmitting/receiving unit 2b. The cancel operation unit 75 is operated, for example, by placing the cursor on the image of the cancel operation unit 75 and clicking or double-clicking it with the mouse. When the cancel operation unit 75 is operated, the information that has been entered up to that point in the input screen 70 is deleted. The user enters information on whether each evaluation item is applicable or unknown for each of all the genetic mutations listed in the first result report on the input screen 70 and operates the setting operation unit 74.

According to the first embodiment, since the undetermined item is displayed on the first result report 51 and the input screen 70, the user can easily grasp the information that needs to be obtained separately and can easily determine the action to be taken. Also, because the user can input evaluation results to the input screen 70 at any time after obtaining additional information, the user can determine the level of pathogenicity after obtaining the additional information, even if a long time is required to obtain the additional information.

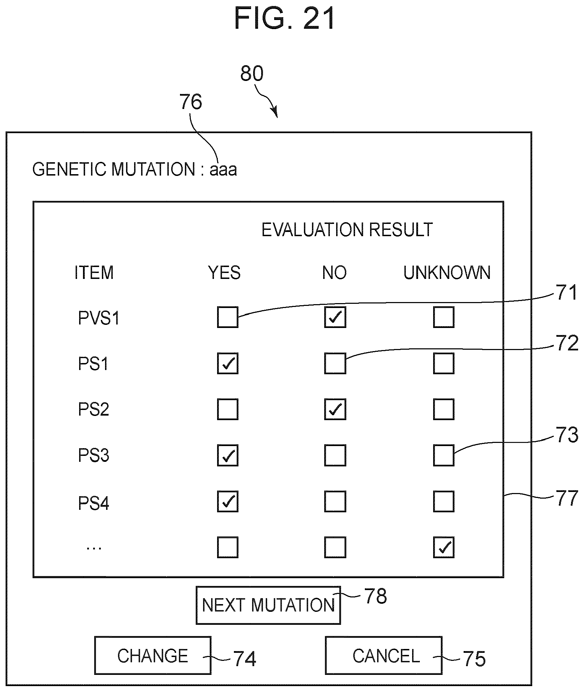

FIG. 21 is a diagram illustrating an input screen 80 of a variation. On the input screen 80, in addition to the evaluation items that are shown to be undetermined in the first result report, an evaluation item that is shown to be determined (the evaluation item listed in the "ACMG Category" in the second region 51b) is also displayed. If the input screen 80 is employed, the user can reconfirm the evaluation items once determined and change the evaluation results as necessary.

Referring again to FIG. 3, in step S15, the control unit 3f of the second report generating device 3 receives each genetic mutation (a genetic mutation displayed in the display area 76 of the input screen 80 (FIG. 20)) and the evaluation result of the evaluation item that is undetermined (the information on whether or not each evaluation item is applicable or unknown, which is entered in the evaluation result display area 77 of the input screen 80 (FIG. 20)) for each of all the genetic mutations listed in the first result report from the data transmitting device 2.

In step S16, for one of the received genetic mutations, the control unit 3f determines the determined evaluation items that are listed in the ACMG category of the first result report 51 and the evaluation items for which the check box 71 of "YES" is checked in the evaluation items received in step S15 as applicable and determines the level of pathogenicity based on the evaluation items determined as applicable and the second criterion, as with the process in step S46 (FIG. 4). When there is no evaluation item for which the check box 71 of "YES" is checked, the control unit 3f may use the level of pathogenicity described in the first result report 51 as the level of pathogenicity of the genetic mutation.

In step S17, as with the process in step S27, the control unit 3f determines whether the level of pathogenicity generated in step S16 is tentative or not. Specifically, the control unit 3f determines whether the level of pathogenicity is changed from the level determined in step S16 when the evaluation result received in step S15, which is "unknown" as to whether it is applicable or not, is changed to "YES" and the level of pathogenicity is determined again. When the level of pathogenicity changes, the control unit 3f determines that the level of pathogenicity is tentative. When the level of pathogenicity does not change, the control unit 3f determines that the level of pathogenicity is not tentative. The control unit 3f also determines that the level of pathogenicity is not tentative when there is no undetermined item.

In step S18, the control unit 3f determines whether the level of pathogenicity is determined for all the genetic mutations received in step S15 by the process in step S16. When a negative judgment is made in step S18, the control unit 3f repeats the processes from step S16 to step S17 until the level of pathogenicity is determined for all the genetic mutations. When a positive judgment is made in step S18, in step S19, the control unit 3f generates a second result report based on the level of pathogenicity determined in step S16 and the tentative or not tentative information determined in step S17. The same format for the second result report can be used as that of the first result report 51 described using FIG. 12A. In step S20, the control unit 3f sends the second result report as an electronic file to the data transmitting device 2 via the transmitting/receiving unit 3c. When step S20 is completed, the process of the control unit 3f is terminated.

In step S4, the control unit 2f of the data transmitting device 2 receives the second result report from the second report generating device 3 and stores it in the memory unit 2g. This allows the physician in charge of the subject to display and view the second result report stored in the memory unit 2g on the display unit 2b at any time.

FIG. 12B is a diagram illustrating an example of a second result report 51' of the first embodiment. This example indicates that, among the evaluation items that are undetermined in the first result report 51, evaluation results of PS2, PM6, PP4, and BS4 are determined, and in step S16 (FIG. 3), for the genetic mutation aaa, the level of pathogenicity is changed from Pathogenic (FIG. 12A) to uncertain significance, and for the genetic mutation aaa, the level of pathogenicity is changed from Likely Pathogenic (FIG. 12A) to Pathogenic. The example also indicates that in step S17 (FIG. 3), the levels of pathogenicity for the genetic mutation aaa and the genetic mutation bbb are determined to be not tentative, and the level of pathogenicity for the genetic mutation ccc is determined to be tentative.

In addition, it is possible that additional information, such as information on the subject's blood relatives, disease patient cohort data, etc., may be obtained by the user after the data transmitting device 2 receives the second result report. In this case, by the control unit 2f of the data transmitting device 2 performing steps S3 to S4 and the control unit 3f of the second report generating device 3 performing steps S15 to S20, the second result report reflecting the additionally obtained information can be generated again.

### Effects of a first embodiment

As described above, according to a report generating method of a first embodiment and a report generation method, since the first result report and the second result report are generated according to the result of determining whether the level of pathogenicity is tentative or not, it is possible to generate a report that can provide appropriate information even when the determined level of pathogenicity may later be changed. This allows the user to easily understand that the level of pathogenicity may change.

### Variation 1

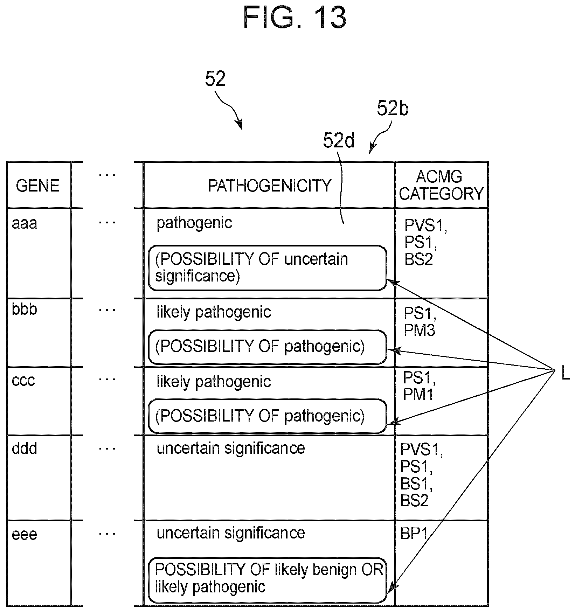

FIG. 13 is a diagram illustrating a partial view of a second region 52b of a first result report 52 (or a second result report) of a variation 1. Description formats of the first and third regions of the first result report 52 of the variation 1 are the same as those in the first result reports 51 and 51' so that the descriptions are omitted.

As illustrated in FIG. 13, when the level of pathogenicity is tentative, the first result report 52 indicates that the level of pathogenicity is tentative by stating the level of pathogenicity after the change, instead of stating a symbol indicating that the level of pathogenicity is tentative. In the first result report 52, in the space indicated by the reference number L in cell 52d where the level of pathogenicity is indicated, the level of pathogenicity after the change is shown in parentheses.

In the example illustrated in FIG. 13, the level of pathogenicity corresponding to the genetic mutation "aaa" is pathogenic, and "(possibility of uncertain significance)" is described under the pathogenic. From this description, the viewer of the first result report 52 can see that there is a possibility that the level of pathogenicity of the genetic mutation "aaa" is changed from pathogenic to uncertain significance later. The example illustrated in FIG. 13 indicates that there is a possibility that the levels of pathogenicity of the genetic mutations "bbb" and "ccc" are changed from likely pathogenic to pathogenic later. Also, it is indicated that there is a possibility that the level of pathogenicity of a genetic mutation "eee" is changed from uncertain significance to likely pathogenic or likely benign. In addition, a level of pathogenicity is not described in parentheses under uncertain significance, which is the level of pathogenicity of the genetic mutation "ddd", which indicates that uncertain significance, which is the level of pathogenicity of the genetic mutation "ddd" is a determined level of pathogenicity.

### Variation 2

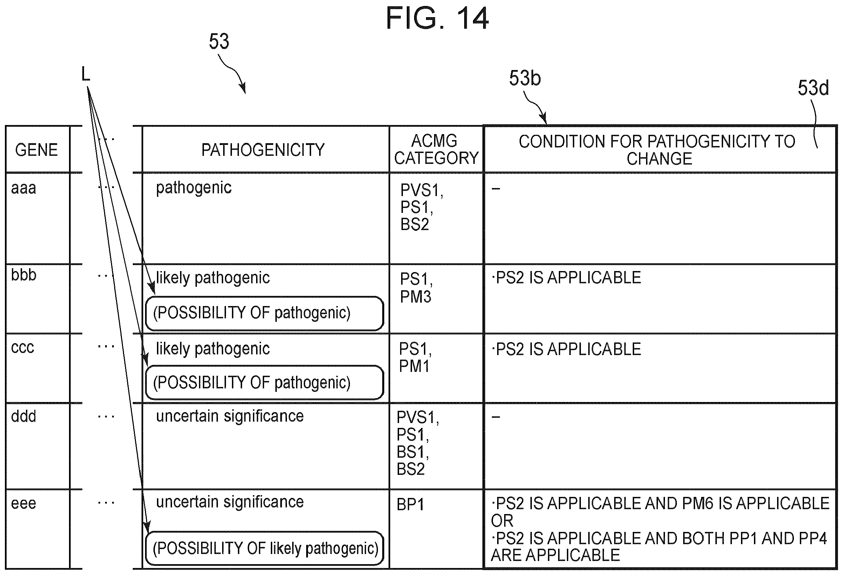

FIG. 14 is a diagram illustrating a partial view of a second region 53b of a first result report 53 (or a second result report) of a variation 2. Description formats of the first and third regions of the first result report 52 of the variation 2 are the same as those in the first result reports 51 and 51' so that the descriptions are omitted.

In the first result report 53 of the variation, when the level of pathogenicity is tentative and the level of pathogenicity after the change is higher than before the change, the level of pathogenicity after the change is described in parentheses in a cell where the level of pathogenicity is indicated. Specifically, as illustrated in FIG. 13, pathogenic, which is the level of pathogenicity of the genetic mutation "aaa", is possible to be changed to uncertain significance later, but the level of pathogenicity of uncertain significance is not higher than that of pathogenic. Therefore, in this case, in the first result report 53, uncertain significance is not indicated under pathogenic in the pathogenicity column of the genetic mutation "aaa".

In contrast, as illustrated in FIG. 13, likely pathogenic, which is the level of pathogenicity of the genetic mutations "bbb" and "ccc" is possible to be changed to pathogenic later. The level of pathogenicity of pathogenic is higher than that of likely pathogenic; therefore, in the first result report 53, "(possibility of pathogenic)" is described under likely pathogenic in the pathogenicity column for the genetic mutation "aaa". Also, when the level of pathogenicity of uncertain significance could be changed to likely pathogenic or likely benign, as in the case of the genetic mutation "eee", only likely pathogenic, whose level of pathogenicity is higher than that of uncertain significance before change, is described as (possibility of likely pathogenic) in parentheses, and likely benign, whose level of pathogenicity is lower than that of uncertain significance before change, is not described.

Thus, by indicating that the level of pathogenicity is tentative when the level of pathogenicity after the change becomes higher than before the change, the possibility of a later change in the direction of a higher level of pathogenicity, which is particularly important in clinical practice, can be easily ascertained.

Furthermore, in the first result report 53 of the variation 2, in the second region 53b, a column 53d for "Conditions for change in pathogenicity" is added to the right of the "ACMG Category" column. The column 53d indicates a condition for when the level of pathogenicity changes to a higher level. In the example illustrated in FIG. 14, in the "Conditions for change in pathogenicity" column for the genetic mutation "bbb", "PS2 is applicable" is described. This allows the viewer of the first result report 53 to easily see, with respect to the genetic mutation "bbb", that likely pathogenic is changed to pathogenic when the PS2 is newly applicable, and for example, the viewer can easy to determine the action to be taken to conduct a test or investigation that can confirm whether or not PS2 corresponds to the genetic mutation "bbb".

The condition for change in pathogenicity may relate to multiple evaluation items as "PS2 is applicable, and PM6 is applicable OR PS2 is applicable, and PP1 and PP4 both applicable" listed in the column of conditions for change in pathogenicity of the genetic mutation "eee". In this case, the viewer can still easily determine the required action.

### Variation 3

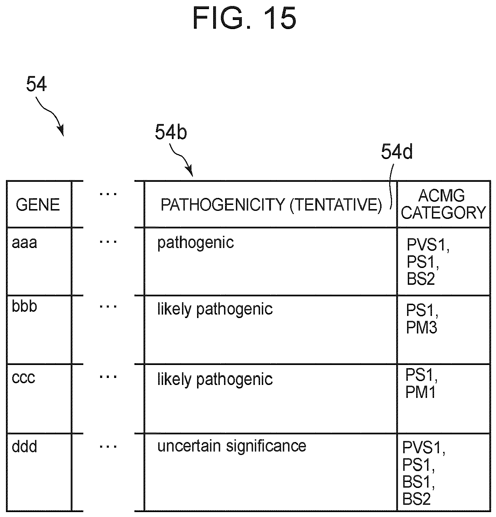

FIG. 15 is a diagram illustrating a partial view of a second region 54b of a first result report 54 (or a second result report) of a variation 3. Description formats of the first and third regions of the first result report 54 of the variation 3 are the same as those in the first result reports 51 and 51' so that the descriptions are omitted.

As illustrated in FIG. 15, the first result report 54 has "(tentative)" to the right of pathogenicity in a column 54d of "Pathogenicity" as an item. This allows the report to indicate that the level of pathogenicity is tentative in a smaller space than when "(tentative)" is listed for each genetic mutation.

### Variation 4

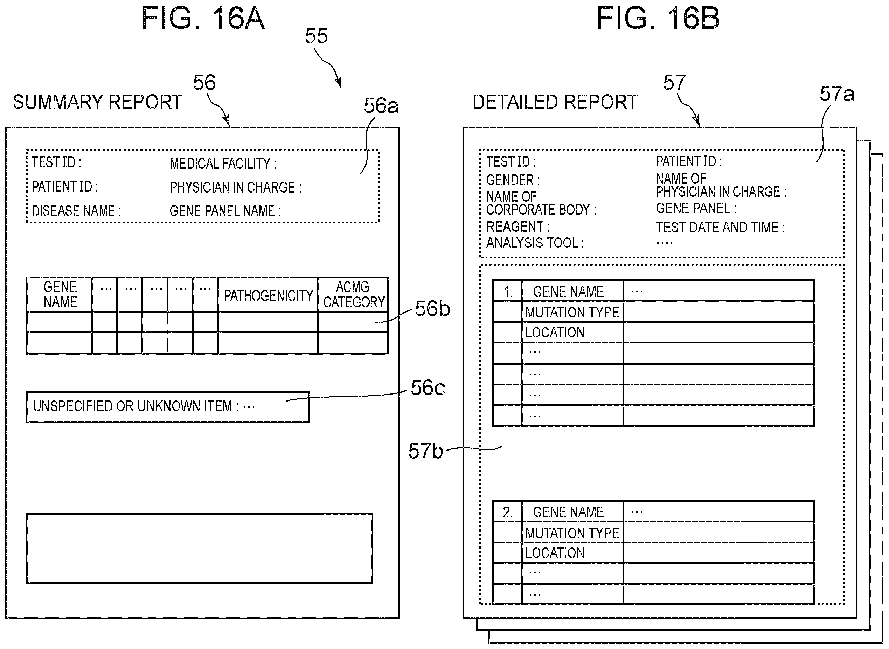

FIG. 16 is a diagram illustrating a first result report 55 (or a second result report) of a variation 4. As illustrated in FIG. 16, the first result report 55 of the variation 4 includes a summary report 56 as the first report and a detailed report 57 as the second report. The summary report 56 is a report for reporting information on a detected genetic mutation and an evaluation result regarding the level of pathogenicity corresponding to the genetic mutation and is mainly viewed by the physician in charge of the subject. The detailed report 57 is a report for reporting information on the detected genetic mutation and the evaluation result regarding the level of pathogenicity corresponding to the genetic mutation and is mainly viewed by each member of an expert panel who interprets a result of a gene panel test. The detailed report 57 reports information regarding the detected genetic mutation in more detail than the summary report 56.

The summary report 56 can adopt the format of the first result report 51 of the first embodiment or the formats of the first result reports 52, 53, 54 of the variations 1 to 3. The summary report 56 consists of a first region 56a, in which a part of the test request information is described, a second region 56b, in which the genetic mutation information, the level of pathogenicity, etc. are described, and a third region 56c, in which an undetermined item is described.

The detailed report 57 includes a first region 57a, in which some or all of the test request information is described, and a second region 57b, in which the genetic mutation information and the level of pathogenicity, etc. are described in more detail than in the second region 56b. In the second region 57b, in addition to the gene name of each mutation, information on the level of pathogenicity, identified evaluation item, genetic format, mutant allele frequency, mutation type (homo or hetero, etc.), base and/or amino acid altered by the mutation, and other details of the mutation are described.

In at least one of the summary report 56 and the detailed report 57, it may be possible to identify an area that describes information about a genetic mutation, for which the level of pathogenicity is determined to be tentative, and the level of pathogenicity corresponding to the information about the genetic mutation and an area that describes information about a genetic mutation, for which the level of pathogenicity is determined not to be tentative, and the level of pathogenicity corresponding to the information about the genetic mutation. For example, an area that describes information about a genetic mutation, for which the level of pathogenicity is determined to be tentative, and the level of pathogenicity corresponding to the information about the genetic mutation and an area that describes information about a genetic mutation, for which the level of pathogenicity is determined not to be tentative, and the level of pathogenicity corresponding to the information about the genetic mutation may be listed in separate tables.

### Variation 5

### (Summary report)

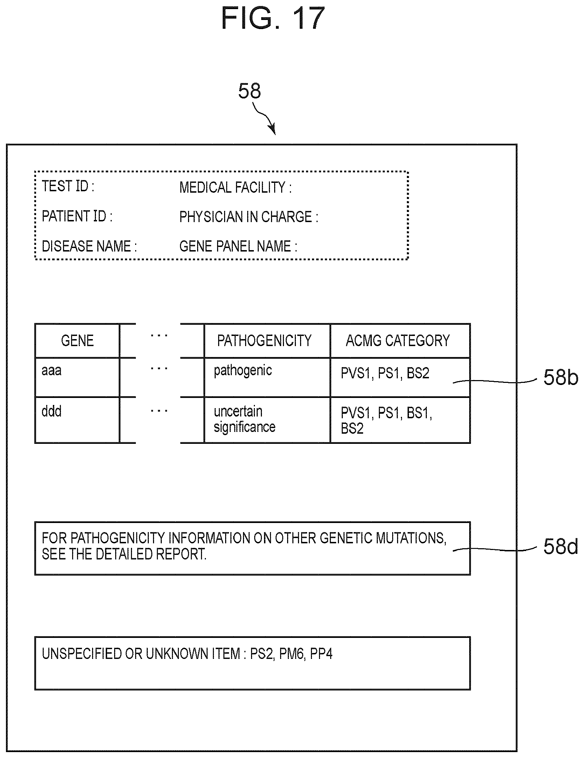

FIG. 17 is a diagram illustrating a summary report 58 of a variation 5. The summary report 58 has the following features and is otherwise identical to the summary report 56. As illustrated in FIG. 17, in a second region 58b of the summary report 58, only the information on a genetic mutation, for which the level of pathogenicity is determined to be not tentative, and the level of pathogenicity corresponding to the information on the genetic mutation are described, while information on a genetic mutation, for which the level of pathogenicity is determined to be tentative, and the level of pathogenicity corresponding to the information on the genetic mutation are not described. When there is a tentative level of pathogenicity, a description, such as "For the level of pathogenicity of other genetic mutations, please refer to the detailed report." Is described in a fourth region 58d, which indicates that there is a tentative level of pathogenicity and where to refer to for the information. This allows the user to quickly ascertain the confirmed level of pathogenicity and to refer to the tentative level of pathogenicity as needed.

### (Detailed report)

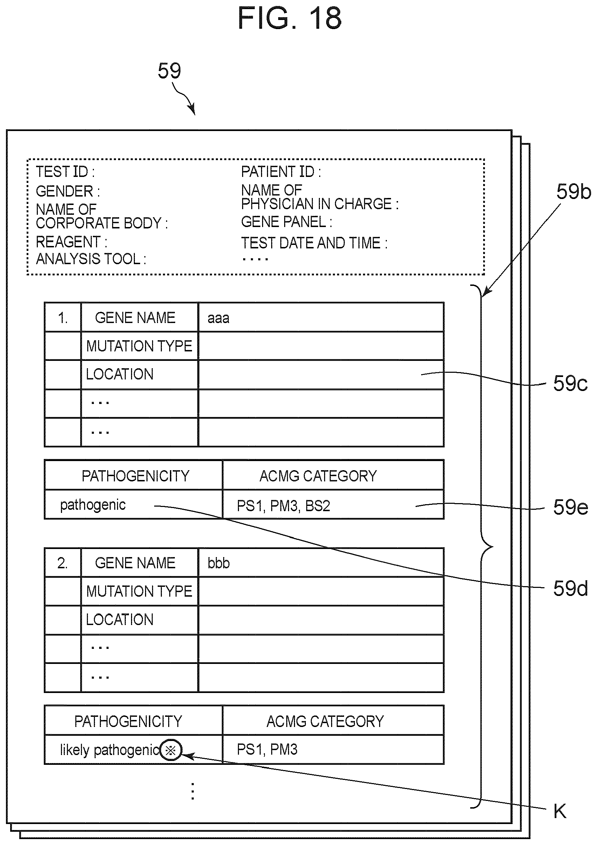

FIG. 18 is a diagram illustrating a detailed report 59 of the variation 5. The detailed report 59 differs from the detailed report 57 only in the description of a second region 59b. The description of a first region 59a of the detailed report 59 is omitted. The second region 59b of the detailed report 59 is divided into two regions, which are a region 59c that describes the genetic mutation information and a region 59d that indicates the level of pathogenicity. The information and the manner in which the information is described in the region 59c and the region 59d may be the same as the information described in the region 57b of the detailed report 57 or may be modified as appropriate.

### Variation 6

Among the levels of pathogenicity, a level of pathogenicity with a relatively high level of pathogenicity may be described in a manner that is distinguishable from other levels of pathogenicity. A relatively high level of pathogenicity is described as "pathogenic" and "likely pathogenic", and a particularly high level of pathogenicity is described as "pathogenic". For example, only information on "pathogenic" and "likely pathogenic" or "pathogenic" and their corresponding genetic mutations may be included in the summary report, with no information on other levels of pathogenicity and their corresponding genetic mutations, and all levels of pathogenicity and their corresponding genetic mutations may be displayed in the detailed report. This allows the summary report to easily identify the level of high pathogenicity, which is particularly important in clinical practice, and to confirm all levels of pathogenicity in the detailed report as needed.

### (Summary report)

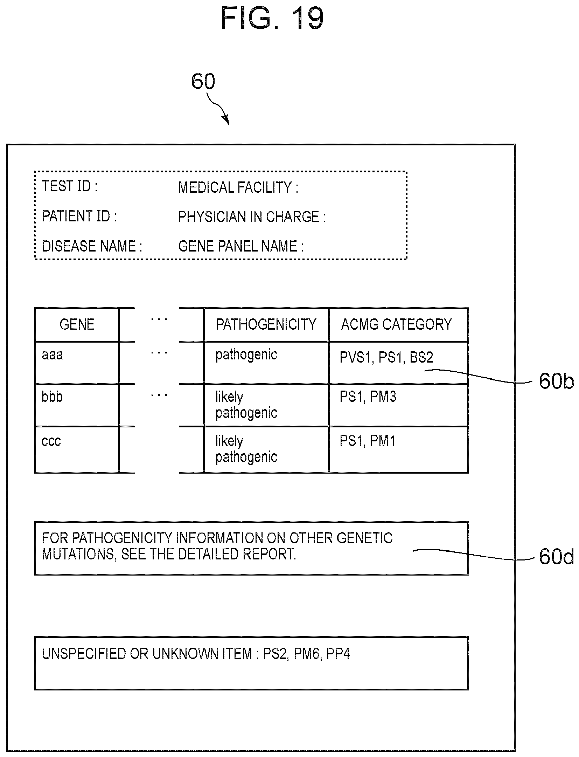

FIG. 19 is a diagram illustrating a summary report 60 of a variation 6. As illustrated in FIG. 19, the summary report 60 displays "pathogenic" and "likely pathogenic", whose levels of pathogenicity are relatively high and do not display other levels of pathogenicity in a second region 60b. In addition, in the second region 60b, only "pathogenic" may be displayed, and other levels of pathogenicity including "likely pathogenic" may not be displayed.

Also, the summary report 60, like the summary report 58, does not describe the level of pathogenicity that is tentative, even if the level of pathogenicity is "pathogenic" or "likely pathogenic". This allows the user to easily ascertain the level of pathogenicity that is relatively high and confirmed. In addition, the manner in which the summary report is described is not limited to those described above; for example, the level of pathogenicity that is tentative may be described in the summary report 60.

### (Detailed report)

A detailed report in the variation 6 can adopt the same report as the detailed report 59 in the variation 5, so the description is omitted. Also, the manner in which the detailed report is described is not limited to those described above; for example, in the detailed report, in addition to the genetic mutation information of the subject, clinical information including the disease name of the subject, genetic information of the subject's blood relatives, and medical history information of the subject's blood relatives may be described.

### Second embodiment

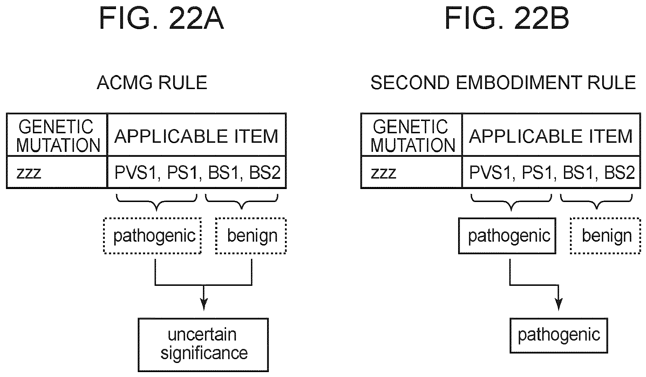

As explained in the procedure for assigning a level of pathogenicity (FIG. 9), as illustrated in FIG. 22A, in the ACMG/AMP guidelines, the level of pathogenicity may be assigned to both "pathogenic" and "benign", in which case the level of pathogenicity is to be determined as "uncertain significance".

Therefore, for example, even if a genetic mutation fits the high level of "pathogenic" from the evaluation results of 16 items of pathogenicity (FIG. 5A), a case occurs in which it is finally determined to be "uncertain significance" because it also fits the level of "benign" from the evaluation results of 12 items of benignancy. In this case, the final determination is "uncertain significance," but it also fits the "pathogenic" category, so the genetic mutation may be an important genetic mutation in the disease the subject has. Based on the above, in a second embodiment, as illustrated in FIG. 22B, the genetic mutation that is applicable to "pathogenic" is determined as "pathogenic" even if the genetic mutation is simultaneously applicable to "benign".

The schematic configuration of a report generating system of a second embodiment is the same as that illustrated in FIG. 1. Also, outlines of processes executed by each control unit of the data transmitting device 2, the second report generating device 3, and the first report generating device 4 are the same as those in a first embodiment, except for the process of assigning the level of pathogenicity, which is described using FIG. 9.

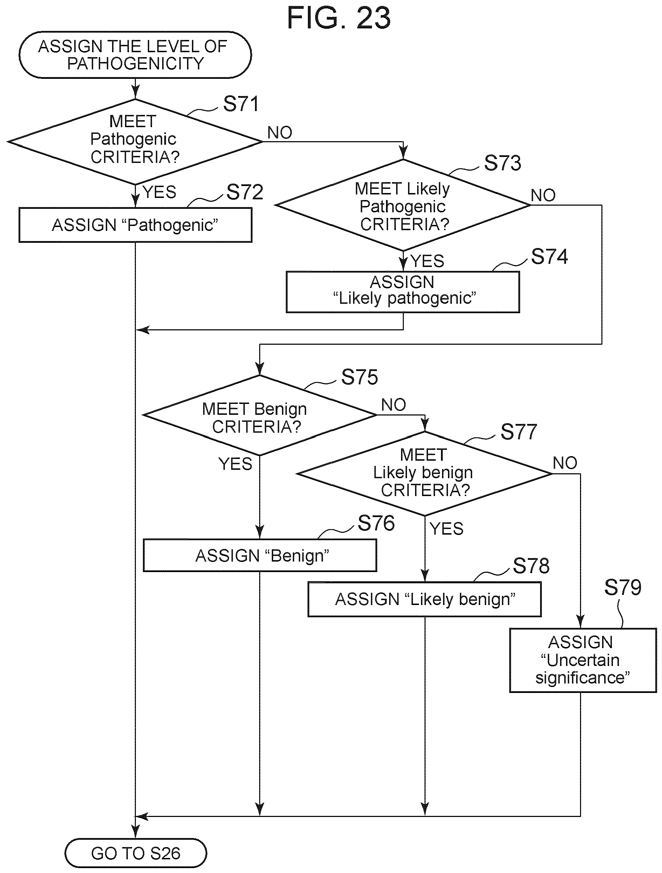

FIG. 23 is a flowchart corresponding to FIG. 9 in a second embodiment and is a flowchart illustrating a procedure for assigning a level of pathogenicity according to a second embodiment. Referring to FIG. 23, when step S45 in FIG. 4 is completed, in step S71, the control unit 4f determines whether 1 genetic mutation detected in step S24 meets the criteria for pathogenic based on the second criteria P(i) to P(iii) (FIG. 8) for pathogenic. When a positive judgment is made in step S71, the control unit 4f assigns pathogenic to the 1 genetic mutation detected in step S24 in step S72 and returns the process to step S26 (FIG. 3).

On the other hand, when a negative judgement is made in step S71, the process moves to step S73, and the control unit 4f determines whether the 1 genetic mutation detected in step S24 meets the criteria for likely pathogenic based on the second criteria LP(i) to LP(vi) for likely pathogenic. When a positive judgment is made in step S73, the control unit 4f assigns likely pathogenic to the genetic mutation in step S74 and returns the process to step S26. On the other hand, when a negative judgement is made in step S73, the control unit 4f moves the process to step S75.

In step S75, the control unit 4f determines whether the genetic mutation meets the criteria for benign based on the second criteria B(i) to B(ii) for benign. When a positive judgement is made in step S75, the control unit 4f assigns benign to the genetic mutation in step S76 and returns the process to step S26.

On the other hand, when a negative judgement is made in step S75, the process moves to step S77, and the control unit 4f determines whether the genetic mutation meets the criteria for likely benign based on the second criteria LB(i) to LB(ii) for likely benign. When a positive judgement is made in step S77, the control unit 4f assigns likely benign to the genetic mutation in step S78 and returns the process to step S26. On the other hand, when a negative judgment is made in step S75, the control unit 4f moves the process to step S79, assigns uncertain significance to the genetic mutation in step S79, and returns the process to step S26.

[Effects of a second embodiment] According to a second embodiment, a user can easily recognize that the detected genetic mutation may be an important genetic mutation in the disease the subject has.

### Third embodiment

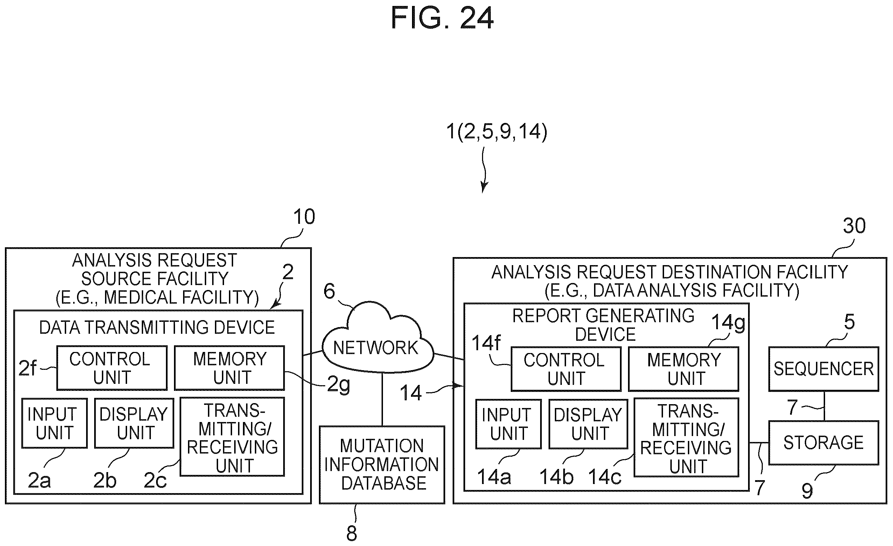

FIG. 24 is a schematic diagram of a report generating system 101 according to a third embodiment. The report generating system 101 is equipped with a data transmitting device 2, a report generating device 14, a sequencer 5, and a storage 9. The data transmitting device 2 and the report generating device 14 are connected to each other via a network 6, which is the internet. A mutation information database 8 is further connected to the network 6. Also, the report generating device 14, the sequencer 5, and the storage 9 are connected to each other via a local area network 7. Hardware configurations of the data transmitting device 2, the report generating device 14, the sequencer 5, and the storage 9 are the same as the data transmitting device 2, the report generating device 14, the sequencer 5, and the storage 9 of a first embodiment, respectively. The data transmitting device 2 sends and receives data to and from the report generating device 14 via the network 6.

The data transmitting device 2 is installed in an analysis request source facility 10, e.g., a hospital (medical facility), a testing center, or a biomedical science laboratory. The report generating device 14 is installed at an analysis request destination facility 30, e.g., a data analysis facility. The analysis request source facility 10 and the analysis request destination facility 30 are different facilities. Also, the analysis request source facility 10 and the analysis request destination facility 30 may be the same facility. The report generating device 14 may be a computer that constitutes a cloud system. The analysis request destination facility 30 may be a facility of a cloud service provider or a facility of a company that provides nucleic acid sequence analysis services. The report generating device 14 is accessible to the mutation information database 8 via the network 6.

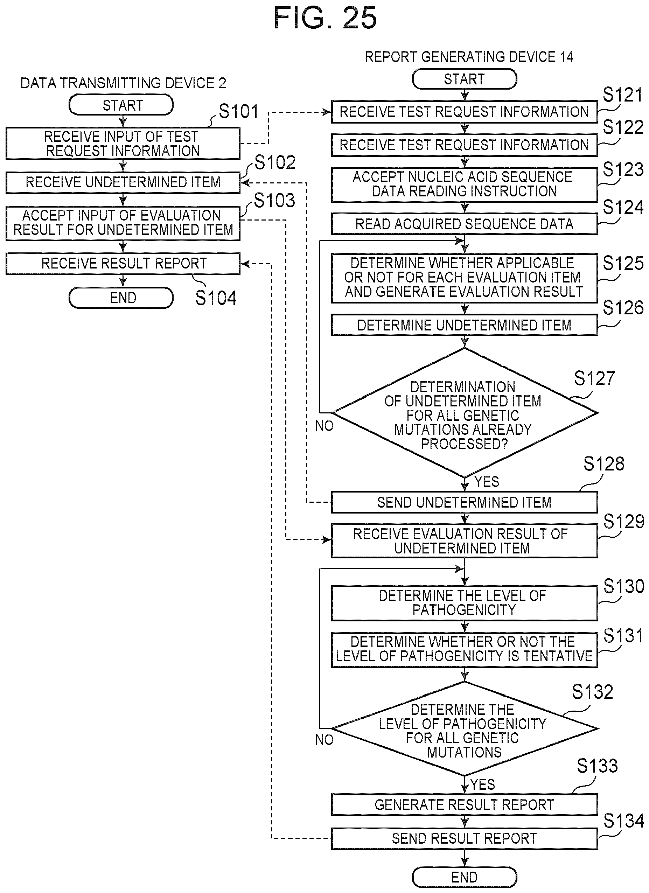

FIG. 25 is a flowchart illustrating processes performed by each control unit of the data transmitting device 2 and the report generating device 14 in a third embodiment. Referring to FIG. 25, in step S101, the control unit 2f of the data transmitting device 2 displays the test request information input screen 40 (see FIG. 2) on the display unit 2b, accepts input of test request information, and transmits the accepted test request information to the report generating device 14. Next, in step S121, the control unit 14f of the report generating device 14 receives the test request information transmitted from the data transmitting device 2, stores it in the memory unit 14g, and displays on the display 14b that the test request information is received.

When a user of the report generating device 14 or a user of the sequencer 5 recognizes that the test request information is accepted by the information displayed on the display unit 14b, the user prepares a library sample from a specimen identified by a specimen ID displayed in the input area 42e of the test request information input screen 40 and has the sequencer 5 read the nucleic acid sequence data. The sequencer 5 records the sequence run data including the read nucleic acid sequence data in the storage 9. When the reading of the nucleic acid sequence data by the sequencer 5 is completed, the user of the report generating device 14 operates the input unit 14a of the report generating device 14 and instructs to read the nucleic acid sequence data. With the instruction, in step S122, the control unit 14f accepts the instruction to read the nucleic acid sequence data by the user of the report generating device 14.

In step S123, the control unit 14f reads the nucleic acid sequence data (hereinafter referred to as "acquired sequence data") identified by the specimen ID displayed in the input area 42e of the test request information input screen 40 from the sequence run data stored in the storage 9. In step S124, the control unit 14f detects all genetic mutations included in the acquired sequence data based on the acquired sequence data and the reference sequence data obtained from the mutation information database 8 and obtains information on the genetic mutation. Also, in step S124, the control unit 14f determines the genetic form, mutant allele frequency, homo or hetero, mutation location (base), and mutation location (amino acid) based on the acquired sequence data and the reference sequence data obtained from the mutation information database 8.

In step S125, the control unit 14f determines whether each of the 28 evaluation items specified by the ACMG/AMP guidelines is applicable and generates an evaluation result based on the first criterion stored in the memory unit 14g and one of the genetic mutations detected in step S124, using the same process as in step S45 (FIG. 4) in the first embodiment. The control unit 14f determines in step S126, by a process similar to step S26 (FIG. 3) of a first embodiment, an undetermined item from each of the evaluation items evaluated in step S125. In step S127, the control unit 14f determines whether the process of determining the undetermined item is performed for all the genetic mutations detected in step S124. When a positive judgment is made in step S127, the control unit 14f proceeds to step S128. When a negative judgment is made in step S127, the control unit 14f returns the process to step S125 and repeats steps S125 to S126 until the process of determining the undetermined item for all genetic mutations is performed. In step S128, the control unit 14f transmits the information on the determined undetermined item to the data transmitting device 2.

In step S102, the control unit 2f of the data transmitting device 2 receives the undetermined item transmitted from the report generating device 14. In step S103, the control unit 2f displays the input screen 80 (FIG. 20 or FIG. 21) on the display unit 2b based on the undetermined item received in step S102, using the same process as in step S3 (FIG. 3) of a first embodiment, accepts an evaluation result (an additional evaluation result) for the undetermined item, and transmits the accepted evaluation result to the report generating device 14. In step S129, the control unit 14f of the report generating device 14 receives the evaluation result of the undetermined item transmitted from the data transmitting device 2. In step S130, the control unit 14f determines the level of pathogenicity for one of the genetic mutations detected in step S124 by a process similar to step S25 (FIG. 3) in a first embodiment.

In step S131, the control unit 14f determines whether the level of pathogenicity determined in step S130 is tentative or not by a process similar to steps S17 and S27 (FIG. 3). In step S132, the control unit 14f determines whether the level of pathogenicity is determined by the process in step S130 for all genetic mutations detected in step S124. When a negative judgement is made in step S132, the control unit 14f repeats the processes from steps S130 to S131 until the level of pathogenicity is determined for all the genetic mutations. When a positive judgement is made in step S132, the control unit 14f generates a result report according to the level of pathogenicity determined in step S130 and the tentative or not tentative information determined in step S131 in step S133. The format of the result report can adopt the same format as the first result report, or the second result report described using FIGS. 12a through 19. In step S134, the control unit 14f sends the result report as an electronic file to the data transmitting device 2 via the transmitting/receiving unit 14c. When step S134 is completed, the process of the control unit 14f ends.

In step S104, the control unit 2f of the data transmitting device 2 receives the result report from the report generating device 14 and stores it in the memory unit 2g. This allows the subject's physician in charge to display and view the result report stored in the memory unit 2g on the display unit 2b at any timing. Also, it is possible that additional information, such as information on the subject's blood relatives, disease patient cohort data, etc., may be obtained by the user after the data transmitting device 2 receives the result report. In this case, by the control unit 2f of the data transmitting device 2 performing processes of steps S102 to S104 and the control unit 14f of the report generating device 14 performing processes steps S129 to S134, a result report reflecting the additionally obtained information can be generated again.

[Effects of a third embodiment] As described above, according to the report generating method according to a third embodiment and the report generating method, since the first result report and the second result report are generated according to the result of determining whether the level of pathogenicity is tentative or not; therefore, it is possible to generate a report that can provide appropriate information even when the determined level of pathogenicity may later be changed. This allows the user to easily understand that the level of pathogenicity may be changed. In addition, according to a third embodiment, the hardware configuration of the system can be simplified because the second report generating device 3 does not exist in comparison with a first embodiment.

### Fourth embodiment

In the above-mentioned first to third embodiments, the report generating devices 3, 4, and 14 determine the undetermined item (preliminary undetermined items) based on the information on the genetic mutation based on the nucleic acid sequence data read from the storage 9 and then receive the evaluation result for the preliminary undetermined item; however, in a fourth embodiment, information for evaluating an undetermined item is included in the test request information, and a result report is generated without determining the preliminary undetermined item. The hardware configuration of a report generating system in a fourth embodiment is the same as that of the report generating system 101 of a third embodiment but may be the same as that of the report generating system 1 of a first embodiment.

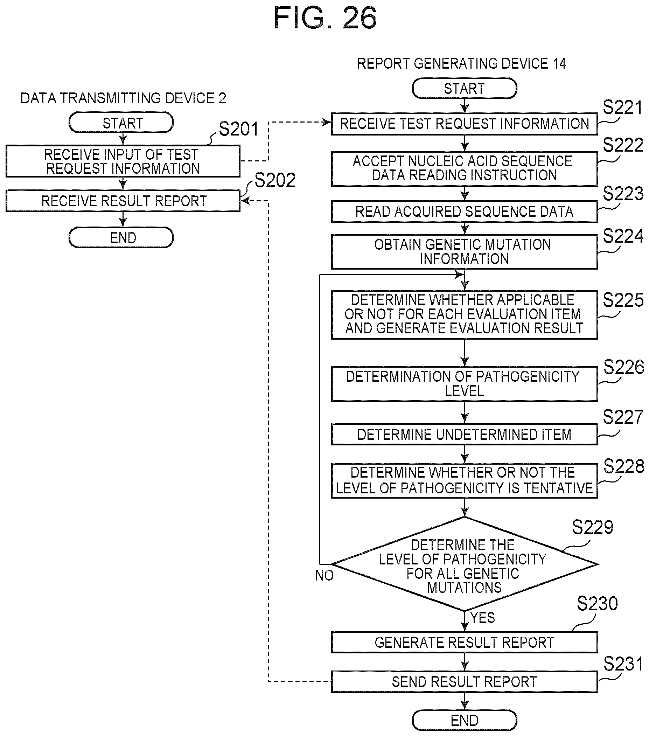

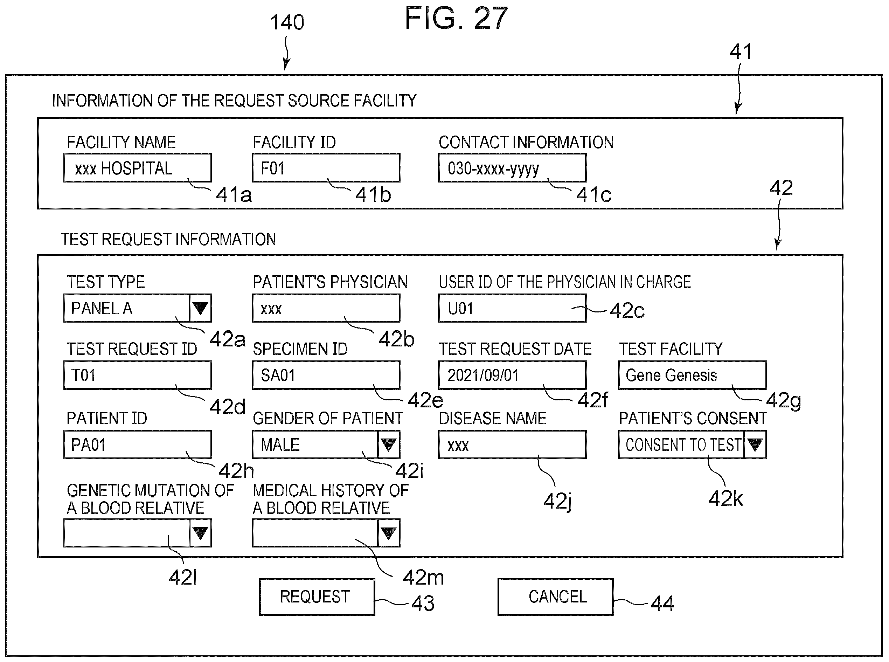

FIG. 26 is a flowchart illustrating processes performed by each control unit of the data transmitting device 2 and the report generating device 14 in a fourth embodiment. Referring to FIG. 26, in step S201, the control unit 2f of the data transmitting device 2 displays a test request information input screen 140 (see FIG. 27) on the display unit 2b, accepts the input of the test request information, and transmits the accepted test request information to the report generating device 14. As illustrated in FIG. 27, in the test request information input screen 140, an input area 421 for inputting a genetic mutation of a blood relative and an input area 42m for inputting the blood relative's medical history are added to the test request information input screen 40 (FIG. 2). The input area 421 is configured in a pull-down list format, and the pull-down list lists genetic mutations of the blood relative associated with the first criterion. The input area 42m is configured in a pull-down list format, and the pull-down list lists the medical history of the blood relative associated with the first criterion. Thus, the test request information of a fourth embodiment includes information on the genetic mutation of the subject's blood relative and information on the medical history of the subject's blood relative. Also, at least one of the input area 421 and the input area 42m can be omitted.

When a user of the report generating device 14 or a user of the sequencer 5 recognizes that the test request information is accepted by the information displayed on the display unit14b, the user prepares a library sample from a specimen identified by a specimen ID displayed in the input area 42e of the test request information input screen 140 and has the sequencer 5 read the nucleic acid sequence data. The sequencer 5 records the sequence run data including the read nucleic acid sequence data in the storage 9. When the reading of the nucleic acid sequence data is completed, the user of the report generating device 14 operates the input unit 14a of the report generating device 14 and gives an instruction to read the nucleic acid sequence data. With the instruction, in step S222, the control unit 14f accepts the instruction to read the nucleic acid sequence data by the user of the report generating device 14.

Referring again to FIG. 26, in step S223, the control unit 14f reads the nucleic acid sequence data (hereinafter referred to as "acquired sequence data") identified by the specimen ID displayed in the input area 42e of the test request information input screen 140 from the sequence run data stored in the storage 9. In step S224, the control unit 14f detects all genetic mutations included in the acquired sequence data based on the acquired sequence data and the reference sequence data obtained from the mutation information database 8 and obtains information on the genetic mutation. Also, in step S224, the control unit 14f determines the genetic form, mutant allele frequency, homo or hetero, mutation location (base), and mutation location (amino acid) based on the acquired sequence data and the reference sequence data obtained from the mutation information database 8.

In step S225, the control unit 14f determines whether or not each of the 28 evaluation items specified by the ACMG/AMP guidelines is applicable and generates evaluation results based on the first criterion stored in the memory unit 14g, information on the genetic mutation, information on the genetic mutation of the subject's blood relative included in the test request information received in step S221, and information on the medical history of the subject's blood relative for one of the genetic mutations detected in step S224, by using the same process as in step S45 (FIG. 4) of a first embodiment. In step S226, the control unit 14f determines the level of pathogenicity by performing the same process as in step S46 (FIG. 4) of a first embodiment. In step S227, the control unit 14f determines an undetermined item from each evaluation item evaluated in step S225, by performing the same process as in step S26 (FIG. 3) of a first embodiment. In step S228, the control unit 14f determines whether the level of pathogenicity determined in step S226 is tentative or not, by performing the same process as in steps S17 and S27 (FIG. 3). In step S229, the control unit 14f determines whether the level of pathogenicity is determined for all genetic mutations detected in step S224. When a positive judgement is made in step S229, the control unit 14f proceeds the process to step S230. When a negative judgment is made in step S229, the control unit 14f returns the process to step S225 and repeats the processes of steps S225 through S228 until the level of pathogenicity is determined for all genetic mutations.

In step S230, the control unit 14f generates a result report according to the level of pathogenicity determined in step S226 and information whether it is tentative or not determined in step S228. The format of the result report can adopt the same format as that of the first result report or the second result report described using FIGS. 12a through 19. In step S231, the control unit 14f sends the result report as an electronic file to the data transmitting device 2 via the transmitting/receiving unit 14c. When step S231 is completed, the process of the control unit 14f ends.

In step S202, the control unit 2f of the data transmitting device 2 receives the result report from the report generating device 14 and stores it in the memory unit 2g. This allows the physician in charge of the subject to display and view the second result report stored in the memory unit 2g on the display unit 2b at any time.
In addition, it is possible that additional information, such as information on the subject's blood relatives, disease patient cohort data, etc., may be obtained by the user after the data transmitting device 2 receives the result report. In this case, by the control unit 2f of the data transmitting device 2 sending the additionally obtained information to the report generating device 14 and the control unit 14f of the report generating device 14 performing processes of steps S225 to S231 based on the additional information received from the data transmitting device 2, a result report reflecting the additionally obtained information can be generated again.

### Effects of a fourth embodiment

As described above, according to the report generating method of a fourth embodiment and the report generating method, since the first result report and the second result report are generated according to the result of determining whether the level of pathogenicity is tentative or not; therefore, it is possible to generate a report that can provide appropriate information even when the determined level of pathogenicity may later be changed. This allows the user to easily understand that the level of pathogenicity may be changed. In addition, according to a fourth embodiment, the software process can be simplified compared to first and third embodiments since there is no process to determine a preliminary undetermined item.

Also, the present invention is not limited to the above-mentioned embodiments and variations thereof, and various improvements and changes are possible within the scope of the claims of the present application and their equivalents. In addition, the report generating method and report generating device of the present invention may be used to generate a report reporting the level of pathogenicity of any disease, for example, a report reporting the level of pathogenicity of a hereditary tumor or a report reporting the level of pathogenicity of a hereditary retinal dystrophy.

For example, in first to third embodiments, in steps S45 (FIG. 4) and S125 (FIG. 25), each of the evaluation items is evaluated based on the information on the genetic mutation of the subject, however, each of the evaluation items may be evaluated based on information contained in the test request information, e.g., the subject's disease information in addition to information on the subject's genetic mutation.

Also, in first to third embodiments, at least one undetermined item for which adding or changing the evaluation result would change the level of pathogenicity is a condition for determining that the level of pathogenicity is tentative; however, the presence of a predetermined number of undetermined items (e.g., one) may be used as a condition for determining that the level of pathogenicity is tentative, regardless of whether or not the level of pathogenicity is changed.

In addition, in first to third embodiments, the second report generating device 3 or the report generating device 14 receives the evaluation result of the undetermined item from the data transmitting device 2; however, the second report generating device 3 or the report generating device 14 may receive additional information, such as information on the genetic mutation of the subject's blood relative, information on the medical history of the subject's blood relative, etc., from the data transmitting device 2, and the control unit of the second report generating device 3 or the report generating device 14 may determine whether or not the undetermined item is applicable based on the received additional information and the first criterion.

Moreover, in first to third embodiments, the first result report 51 and the second result report 51' include the level of pathogenicity and information indicating that the level of pathogenicity is tentative when the level of pathogenicity is determined to be tentative; however, the first result report 51 and/or the second result report 51' may not include the level of pathogenicity when the level of pathogenicity is determined to be tentative. This allows the viewer of the report to easily identify the level of pathogenicity that is not tentative, even if the determined level of pathogenicity may later be changed.

Furthermore, in first to third embodiments, the first result report 51 and the second result report 51' include the level of pathogenicity and the information indicating that the level of pathogenicity is tentative when the level of pathogenicity is determined to be tentative; however, the first result report 51 and/or the second result report 51' may add information indicating that the level of pathogenicity is confirmed to a level of pathogenicity determined not to be tentative, i.e., a confirmed level of pathogenicity. Even in this way, each level of pathogenicity indicated in the first result report 51 and/or the second result report 51' can still be described whether tentative or not.

Also, in first to third embodiments, the first and second criteria based on the ACMG/AMP guidelines or rules that modify those guidelines are used; however, the first and second criteria based on other guidelines may also be used.

## Claims

1. A method of generating a report that reports a level of pathogenicity of a genetic mutation, comprising
obtaining information on a genetic mutation detected in a gene panel test; generating an evaluation result for a plurality of evaluation items based on a first criterion for evaluating each of the evaluation items and at least the information on the genetic mutation;
determining the level of pathogenicity based on a second criterion for determining the level of pathogenicity and the evaluation result;
determining an undetermined item for which the evaluation result is undetermined among the plurality of evaluation items, and determining whether the level of pathogenicity is tentative based on a result of the determination of the undetermined item; and
generating the report that reports the level of pathogenicity according to a result of the determination on whether the level of pathogenicity is tentative.

2. The method of generating a report according to claim 1, wherein
in case that the level of pathogenicity is determined to be tentative, the report comprises the level of pathogenicity and information indicating whether the level of pathogenicity is tentative.

3. The method of generating a report according to claim 2, wherein
in case that the information on the genetic mutation includes a plurality of genetic mutations,
for each of the genetic mutations, the generating the evaluation result, the determining the level of pathogenicity, and the determining whether the level of pathogenicity is tentative are executed,
in case that the level of pathogenicity is determined to be tentative for at least one of the genetic mutations, the report comprises the information indicating whether the level of pathogenicity is tentative, in a manner in which the level of pathogenicity determined to be tentative is identifiable.

4. The method of generating a report according to any one of claims 1 to 3, wherein
the determining whether the level of pathogenicity is tentative comprising determining the level of pathogenicity to be tentative when the result of the determination of the undetermined item meets a predetermined condition.

5. The method of generating a report according to claim 4, wherein
the predetermined condition is that the level of pathogenicity is changed by an evaluation result of at least one of the undetermined items being added to the evaluation result.

6. The method of generating a report according to claim 4, wherein
the predetermined condition is that there are at least a predetermined number of undetermined items.

7. The method of generating a report according to any one of claims 1 to 6, wherein
the report comprises first and second reports that report information on the genetic mutation and information on the level of pathogenicity corresponding to the information on the genetic mutation, wherein
the second report reports the information of the genetic mutation in more detail than the first report.

8. The method of generating a report according to claim 7, wherein
the first report is generated according to the determination result of whether the level of pathogenicity is tentative and the level of pathogenicity.

9. The method of generating a report according to claim 8, wherein
In case that the level of pathogenicity corresponding to the information on the genetic mutation is higher than a predetermined level, the first report comprises the information of the genetic mutation and the level of pathogenicity, and in case that the level of pathogenicity corresponding to the information of the genetic mutation is the predetermined level or lower, the first report does not comprise the information of the genetic mutation and the level of pathogenicity.

10. The method of generating a report according to any one of claims 7 to 9, wherein
in case that the level of pathogenicity corresponding to the information of the genetic mutation is determined to be tentative, the first report does not comprise the information of the genetic mutation and the level of pathogenicity determined to be tentative, and the second report comprises the information of the genetic mutation, the level of pathogenicity determined to be tentative, and information indicating whether the level of pathogenicity is tentative or not.

11. The method of generating a report according to any one of claims 1 to 10, wherein the report comprises information on the genetic mutation.

12. The method of generating a report according to any one of claims 1 to 11, wherein the report comprises information indicating the undetermined item.

13. The method of generating a report according to claim 1, wherein
in case that the level of pathogenicity corresponding to the information of the genetic mutation is determined to be tentative, the report does not comprise the information of the genetic mutation and the level of pathogenicity determined to be tentative.

14. The method of generating a report according to any one of claims 1 to 13, wherein the determining the undetermined item comprising:
determining a preliminary undetermined item from the plurality of evaluation items based on information including the information on the genetic mutation;
generating the evaluation result for the preliminary undetermined item; and
determining the undetermined item from the preliminary undetermined item based on the evaluation result of the preliminary undetermined item.

15. The method of generating report according to claim 14, wherein
the generating the evaluation result for the preliminary undetermined item comprising:
providing a screen to a user for accepting an evaluation result for the preliminary undetermined item; and
accepting input of the evaluation result for the preliminary undetermined item via the screen.

16. The method of generating a report according to claim 14, wherein
the generating the evaluation result for the preliminary undetermined item comprising:
obtaining additional information to evaluate the preliminary undetermined item; and
determining the evaluation result for the preliminary undetermined item based on the obtained additional information and the first criterion.

17. The method of generating a report according to one of claims 1 to 16, further comprising
obtaining clinical information including disease information of a subject, wherein the generating the evaluation result comprises generating the evaluation result based on the first criterion, the information on the genetic mutation, and the clinical information.

18. The method of generating a report according to claim 17, further comprising
obtaining at least one of genetic information of a blood relative of the subject and medical history information of the blood relative of the subject, wherein
the generating the evaluation result comprises generating the evaluation result based on the first criterion, the information on the genetic mutation, the clinical information, and at least one of the genetic information and medical history information of the blood relative of the subject.

19. The method of generating a report according to any one of claims 1 to 18, wherein the first criterion comprising a criterion relating to at least one the following:
a disease name;
information of at least one of a type of a genetic mutation, a location of the genetic mutation, and at least one of amino acids and bases altered by the mutation;
information comprising at least one of genetic information and medical history of a blood relative;
information on association between a disease and a genetic mutation;
a frequency of a genetic mutation in at least one of a control population and a disease population;
region information for each gene; and
information obtained by a prediction algorithm.

20. The method of generating a report according to any one of claims 1 to 19, wherein
the obtaining the information on the genetic mutation comprises obtaining the information on the genetic mutation via a network from a storage device that stores the information on the genetic mutation.

21. A report generating device that generates a report for reporting a level of pathogenicity of a genetic mutation, comprising:
a controller; and
an output device, wherein
the controller is programmed to
obtain information on a genetic mutation detected in a gene panel test;
generate an evaluation result for a plurality of evaluation items based on a first criterion for evaluating each of the evaluation items and at least the information on the genetic mutation;
determine the level of pathogenicity based on a second criterion for determining the level of pathogenicity and the evaluation result;
determine an undetermined item for which the evaluation result is undetermined among the plurality of evaluation items, and determine whether the level of pathogenicity is tentative based on a result of the determination of the undetermined item; and
generate the report that reports the level of pathogenicity according to a result of the determination on whether the level of pathogenicity is tentative, and wherein
the output unit outputs the report.
